# EUROPEAN PATENT APPLICATION

(11) **EP 1 894 923 A1**
(43) Date of publication of application: **05.03.2008**
(21) Application number: 06731873.3
(22) Date of filing: 14.04.2006
(51) Int. Cl.: C07D 333/76, C09K 11/06, H01L 51/50

(54) **BENZOTHIOPHENE DERIVATIVE AND ORGANIC ELECTROLUMINESCENCE DEVICE MAKING USE OF THE SAME**

(30) Priority: 24.06.2005 JP 2005184506
(71) Applicant: IDEMITSU KOSAN CO., LTD., Tokyo 100-8321 (JP)
(72) Inventor: ITO, Mitsunori, 2990293 (JP); KUBOTA, Mineyuki, 2990293 (JP); HOSOKAWA, Chishio, 2990293 (JP)
(74) Representative: Gille Hrabal Struck Neidlein Prop Roos
(86) International application number: PCT/JP2006/307937
(87) International publication number: WO 2006/137210

(57) **Abstract**

A benzothiophene derivative that having novel structure and an electroluminescence device which comprises a cathode, an anode and an organic thin film layer which comprises at least one layer comprising at least a light emitting layer and is disposed between the cathode and the anode, wherein at least one layer in the organic thin film layer comprises the benzothiophene derivative described in Claim 1 singly or as a component of a mixture. An electroluminescence device emitting blue light, exhibiting a great efficiency of light emission and having a long life can be obtained by using the derivative.

## Description

### TECHNICAL FIELD

The present invention relates to a benzothiophene derivative and an organic electroluminescence (EL) device utilizing the derivative. More particularly, the present invention relates to an organic EL device emitting blue light, exhibiting a great efficiency of light emission and having a long life and a benzothiophene derivative providing the device.

### BACKGROUND ART

An organic EL device is a spontaneous light emitting device which utilizes the principle that a fluorescent substance emits light by energy of recombination of holes injected from an anode and electrons injected from a cathode when an electric field is applied. Since an organic EL device of the laminate type driven under a low electric voltage was reported by C. W. Tang of Eastman Kodak Company (C. W. Tang and S. A. Vanslyke, Applied Physics Letters, Volume 51, Pages 913, 1987), many studies have been conducted on organic EL devices using organic materials as the constituting materials. Tang et al. used tris(8-hydroxyquinolinolato)-aluminum for the light emitting layer and a triphenyldiamine derivative for the hole transporting layer. Advantages of the laminate structure are that the efficiency of hole injection into the light emitting layer can be increased, that the efficiency of forming excited particles which are formed by blocking and recombining electrons injected from the cathode can be increased, and that excited particles formed within the light emitting layer can be enclosed. As the structure of the organic EL device, a two-layered structure having a hole transporting (injecting) layer and an electron transporting and light emitting layer and a three-layered structure having a hole transporting (injecting) layer, a light emitting layer and an electron transporting (injecting) layer are well known. To increase the efficiency of recombination of injected holes and electrons in the devices of the laminate type, the structure of the device and the process for forming the device have been studied.

As the light emitting material, chelate complexes such as tris(8-quinolinolato)aluminum complex, coumarine derivatives, tetraphenylbutadiene derivatives, bisstyrylarylene derivatives and oxadiazole derivatives are known. It is reported that light in the visible region ranging from blue light to red light can be obtained by using these light emitting materials, and development of a device exhibiting color images is expected (for example, Patent References 1 to 3).

Recently, many studies using a phosphorescent compound as the light emitting material have been conducted so that the energy of the triplet state is utilized for the EL light emission. For example, it is reported by a group of Princeton University that an organic EL device using a iridium complex compound as the light emitting material exhibits a great efficiency of light emission (Non-Patent Reference 1). Besides the organic EL devices using a low molecular weight compound such as those described above, an organic EL device using a conjugated macromolecule is reported by a group of Cambridge University (Non-Patent Reference 2). In this report, a film is formed with polyphenylenevinylene (PPV) in accordance with a coating process, and it is confirmed that light is emitted from the film having a single layer.

The recent progress in the organic light emitting device is remarkable and is characterized in that light emitting devices providing a greater luminance under a lower applied voltage and a greater variety of the wavelength of the emitted light, exhibiting a faster response and having a smaller thickness and a smaller weight can be developed. Therefore, it is suggested that the organic light emitting devices can be applied to areas in wide ranges.

As the organic light emitting device makes remarkable progress, requirements for the light emitting material are increasing. In Patent References 4 and 5, pyrene compounds having fluorenylene as the bonding group are disclosed. In Patent Reference 6, pyrene compounds having phenylene or biphenylene as the bonding group are disclosed. However, these compounds have drawbacks in that the half life is insufficient or that the purity of color of the emitted light is poor, and a light emitting material exhibiting a greater luminance of emitted light and a greater efficiency of light emission is desired. A light emitting material which exhibits a smaller change with time during the used for a long time, improved durability with a smaller degradation under an atmosphere of a gas containing oxygen and under moisture and emits blue, green and red light with a greater purity of color with consideration of the application to full color displays is desired.
[Patent Reference 1] Japanese Patent Application Laid-Open No. Heisei 8(1996)-239655
[Patent Reference 2] Japanese Patent Application Laid-Open No. Heisei 7(1995)-183561
[Patent Reference 3] Japanese Patent Application Laid-Open No. Heisei 3(1991)-200289
[Patent Reference 4] Japanese Patent Application Laid-Open No. 2004-83481
[Patent Reference 5] Japanese Patent Application Laid-Open No. 2004-43349
[Patent Reference 6] Japanese Patent Application Laid-Open No. 2004-139957
[Non-Patent Reference 1] Nature, 395, 151 (1998)
[Non-Patent Reference 2] Nature, 347, 539 (1990)

### DISCLOSURE OF THE INVENTION

The present invention has been made to overcome the above problems and has an object of providing an organic EL device emitting blue light, exhibiting a great efficiency of light emission and having a long life and a novel benzothiophene derivative providing the device.

As the result of intensive studies by the present inventors, it was found that the above object could be achieved by using a benzothiophene derivative having the specific structure represented by the following general formula (1) as a material for the organic EL device. The present invention has been completed based on the knowledge.

The present invention provides a benzothiophene derivative represented by following general formula (1):

(A)ₕ-(X)ₖ-(B)ₘ-L-(C)ₙ-(Y)ₚ-(D)_{q} (1)

wherein
X represents a substituted or unsubstituted pyrene residue group;
A and D each independently represent hydrogen atom, a substituted or unsubstituted aromatic hydrocarbon group having 3 to 50 ring carbon atoms, a substituted or unsubstituted aromatic heterocyclic group having 1 to 50 ring carbon atoms, a substituted or unsubstituted alkyl or alkylene group having 1 to 50 carbon atoms or a substituted or unsubstituted alkenyl or alkenylene group having 1 to 50 carbon atoms;
B and C each independently represent a single bond, a substituted or unsubstituted aromatic hydrocarbon group having 3 to 50 ring carbon atoms, a substituted or unsubstituted aromatic heterocyclic group having 1 to 50 ring carbon atoms, a substituted or unsubstituted alkyl or alkylene group having 1 to 50 carbon atoms or a substituted or unsubstituted alkenyl or alkenylene group having 1 to 50 carbon atoms;
Y represents a substituted or unsubstituted condensed cyclic group and/or condensed heterocyclic group having 5 to 50 ring carbon atoms;
L represents a substituted or unsubstituted benzothiophenylene group;
**k** represents an integer of 1 to 3;
h and q each represent an integer of 0 to 4;
m and n each represent an integer of 0 to 5; and
p represents an integer of 0 to 3.

The present invention also provides an electroluminescence device which comprises a cathode, an anode and an organic thin film layer which comprises at least one layer comprising at least a light emitting layer and is disposed between the cathode and the anode, wherein at least one layer in the organic thin film layer comprises the benzothiophene derivative described in Claim 1 singly or as a component of a mixture.

### THE EFFECT OF THE INVENTION

The organic EL device using the benzothiophene derivative described above emits blue light, exhibits a great efficiency of light emission and has a long life.

### THE MOST PREFERRED EMBODIMENT TO CARRY OUT THE INVENTION

The benzothiophene derivative of the present invention is represented by the following general formula (1):

(A)ₕ-(X)ₖ-(B)ₘ-L-(C)ₙ-(Y)ₚ-(D)_{q} (1)

In general formula (1), **k** represents an integer of **1** to 3, **h** and **q** each represent an integer of 0 to 4, m and n each represent an integer of 0 to 5, and **p** represents an integer of 0 to 3.

In general formula (1), X represents a substituted or unsubstituted pyrene residue group. Examples of the substituted or unsubstituted pyrene residue group include residue groups obtained by removing hydrogen atom from the following structures:

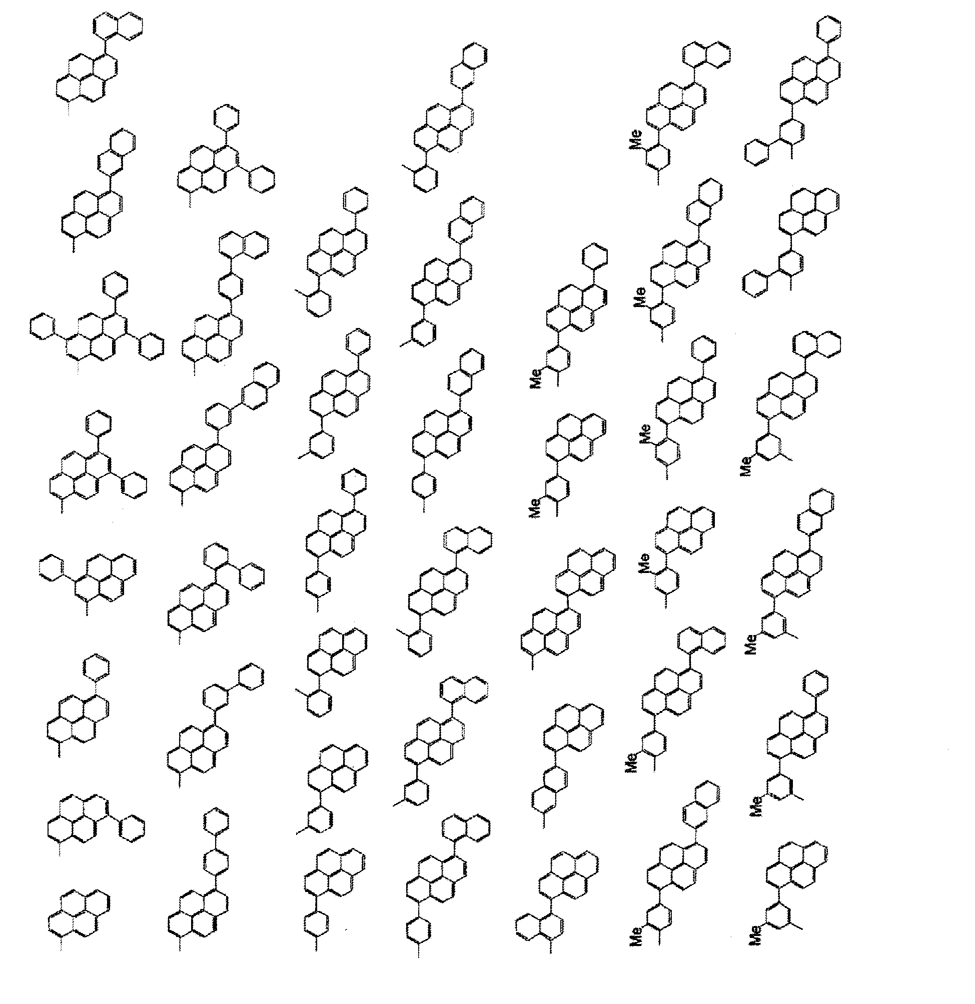

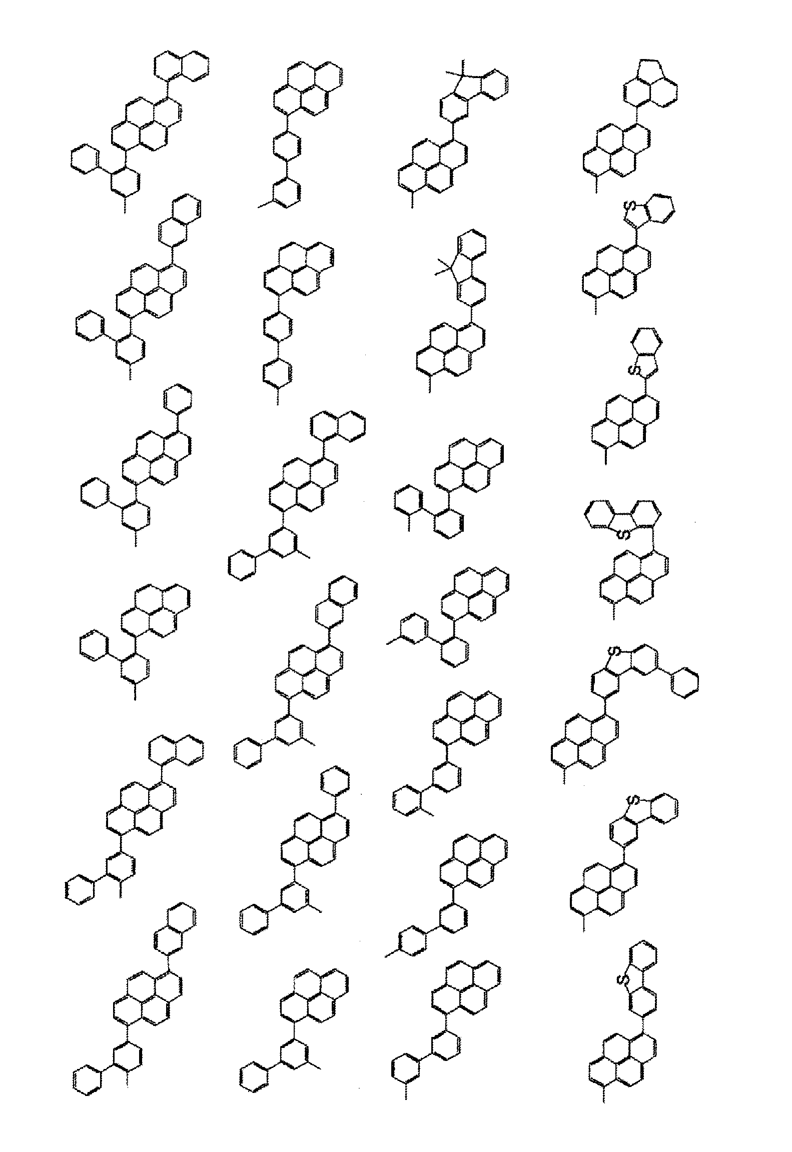

In general formula (1), A and D each independently represent hydrogen atom, a substituted or unsubstituted aromatic hydrocarbon group having 3 to 50 ring carbon atoms, a substituted or unsubstituted aromatic heterocyclic group having 1 to 50 ring carbon atoms, a substituted or unsubstituted alkyl or alkylene group having 1 to 50 carbon atoms or a substituted or unsubstituted alkenyl or alkenylene group having 1 to 50 carbon atoms.

B and C each independently represent a single bond, a substituted or unsubstituted aromatic hydrocarbon group having 3 to 50 ring carbon atoms, a substituted or unsubstituted aromatic heterocyclic group having 1 to 50 ring carbon atoms, a substituted or unsubstituted alkyl or alkylene group having 1 to 50 carbon atoms or a substituted or unsubstituted alkenyl or alkenylene group having 1 to 50 carbon atoms;

Examples of the aromatic hydrocarbon group described above include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1 naphthyl group, 4-methyl-1-anthryl group, 4'-methylbiphenylyl group, 4"-t-butyl-p- terphenyl-4-yl group and divalent groups derived from these groups.

Examples of the aromatic heterocyclic group described above include 1-pyrrolyl group, 2-pyrrolyl group, 3-pyrrolyl group, pyradinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxanyl group, 5-quinoxanyl group, 6-quinoxanyl group, 1-carbazolyl group, 2-carbazolyl group, 3-carbazolyl group, 4-carbazolyl group, 9-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1, 10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 10-phenothiazinyl group, 1-phenoxazinyl group, 2-phenoxazinyl group, 3-phenoxazinyl group, 4-phenoxazinyl group, 10-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methyl-pyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, 4-t-butyl-3-indolyl group and divalent groups derived from these groups.

Examples of the alkyl or alkylene group described above include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitro-ethyl group, 1,3-dinitroisopropyl group, 2,3-dinitro-t-butyl group, 1,2,3-trinitropropyl group and divalent groups derived from these groups.

Examples of the alkenyl or alkenylene group described above include vinyl group, allyl group, 1-propenyl group, 1-butenyl group, 2-butenyl group, 3-butenyl group, 1,3-butadienyl group, 1-methylvinyl group, styryl group, 2,2-diphenylvinyl group, 1,2-diphenylvinyl group, 1-methylallyl group, 1,1-dimethylallyl group, 2-methylallyl group, 1-phenylallyl group, 2-phenylallyl group, 3-phenylallyl group, 3,3-diphenylallyl group, 1,2-dimethylallyl group, 1-phenyl-1-butenyl group, 3-phenyl-1-butenyl group and divalent groups derived from these groups.

Specific examples of the group represented by B and C include the following structures. However, the group represented by B and C is not limited to the groups shown as the examples.

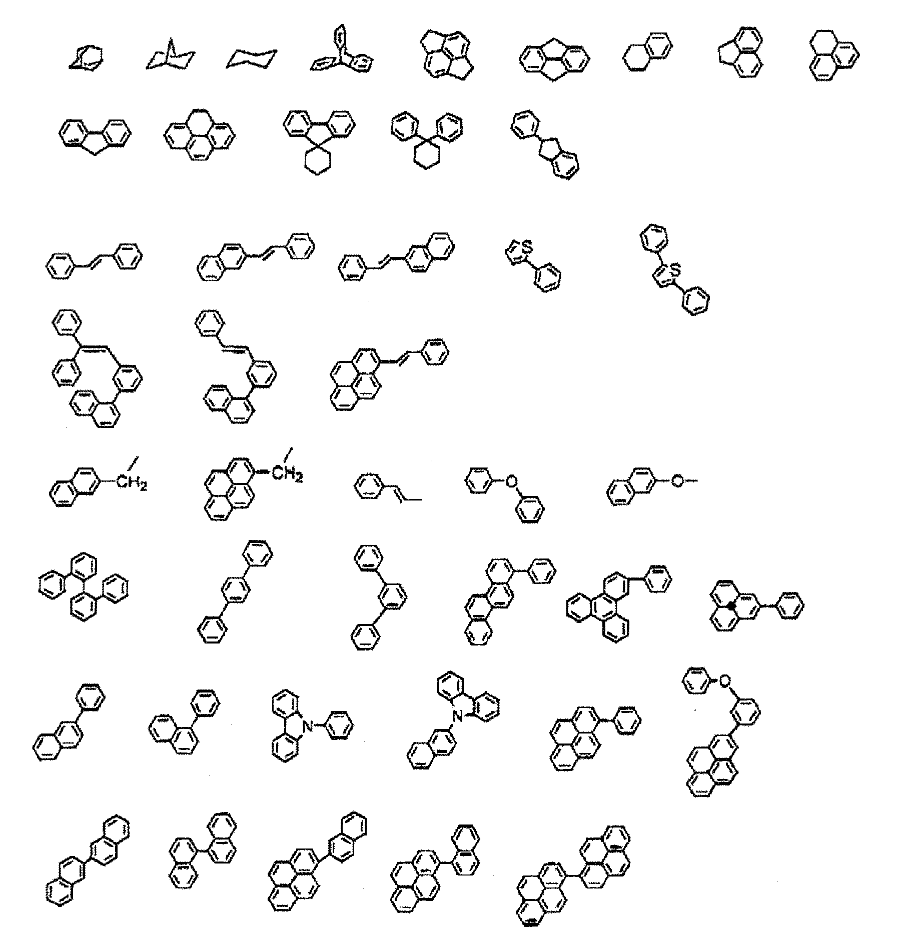

In general formula (1), Y represents a substituted or unsubstituted condensed cyclic group and/or condensed heterocyclic group having 5 to 50 ring carbon atoms. Examples of the group represented by Y include residue groups derived from pyrene, anthracene, benzanthracene, naphthalene, fluoranthene, fluorene, benzofluorene, diazafluorene, phenanthrene, tetracene, coronene, chrysene, fluorescein, perylene, phthaloperylene, naphthaloperylene, perynone, phthaloperynone, naphthaloperynone, diphenylbutadiene, tetraphenylbutadiene, coumarine, oxadiazole, aldazine, bisbenzoxazoline, bistyryl, pyrazine, phenanthroline, quinazoline, cyclopentadiene, imine, diphenylethylene, vinylanthracene, diaminocarbazole, pyrane, thiopyrane, polymethine, melocyanine, oxinoid compounds chelated with imidazole, quinacridone, rubrene, stilbene-based derivatives and fluorescent coloring agents. Among these groups, residue groups derived from pyrene, anthracene and fluoranthene are preferable.

In general formula (1), L represents a substituted or unsubstituted benzothiophenylene group. Examples of the group represented by L include groups represented by general formulae (4) to (7), and benzothiophenylene group and dibenzothiophenylene group are preferable.

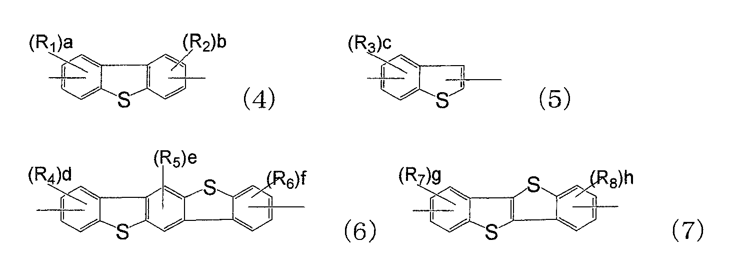

In general formulae (4) to (7), a to d and f to h each represent an integer of 0 to 3, and e represents an integer of 0 to 2.

In general formulae (4) to (7), R₁ to R₈ each independently represent a substituted or unsubstituted aromatic hydrocarbon group having 3 to 50 ring carbon atoms, a substituted or unsubstituted aromatic heterocyclic group having 1 to 50 ring carbon atoms, a substituted or unsubstituted alkyl group having 1 to 50 carbon atoms, a substituted or unsubstituted alkoxyl group having 1 to 50 carbon atoms, a substituted or unsubstituted aralkyl group having 1 to 50 carbon atoms, a substituted or unsubstituted aryloxyl group having 1 to 50 carbon atoms, a substituted or unsubstituted arylthio group having 1 to 50 carbon atoms, a substituted or unsubstituted alkoxycarbonyl group having 1 to 50 carbon atoms, carboxyl group, a halogen atom, cyano group, nitro group or hydroxyl group. Adjacent groups represented by any of R₁ to R₈ may be bonded to each other to form a cyclic structure when a plurality of groups are present.

Examples of the aromatic hydrocarbon group, the aromatic heterocyclic group and the alkyl group include the groups described above as the examples of the corresponding groups.

The alkoxyl group is a group represented by -OY. Examples of the group represented by Y include the group described above as the examples of the alkyl group.

Examples of the aralkyl group include benzyl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, 2-β-naphthylisopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group and 1-chloro-2-phenylisopropyl group.

The aryloxyl group is a group represented by -OZ. Examples of the group represented by Z include the groups described above as the examples of the aromatic hydrocarbon group and the aromatic heterocyclic group.

The arylthio group is a group represented by -SZ. Examples of the group represented by Z include the groups described above as the examples of the aromatic hydrocarbon group and the aromatic heterocyclic group.

The alkoxycarbonyl group is a group represented by -COOY. Examples of the group represented by Y include the group described above as the examples of the alkyl group.

Examples of the halogen atom include fluorine atom, chlorine atom, bromine atom and iodine atom.

Examples of the cyclic structure which may be formed include structures of cycloalkanes having 4 to 12 carbon atoms such as cyclobutane, cyclopentane, cyclohexane, adamantane and norbornane, structures of cycloalkenes having 4 to 12 carbon atoms such as cyclobutene, cyclopentene, cyclohexene, cycloheptene and cyclooctene, structures of cycloalkadienes having 6 to 12 carbon atoms such as cyclohexadiene, cycloheptadiene and cyclooctadiene, aromatic rings having 6 to 50 carbon atoms such as benzene ring, naphthalene ring, phenanthrene ring, anthracene ring, pyrene ring, chrysene ring and acenaphthylene ring and hetero-rings having 5 to 50 carbon atoms such as imidazole ring, pyrrol ring, furan ring, thiophene ring and pyridine ring.

It is preferable that the group represented by L is a group expressed by any one of the following structures among the above structures.

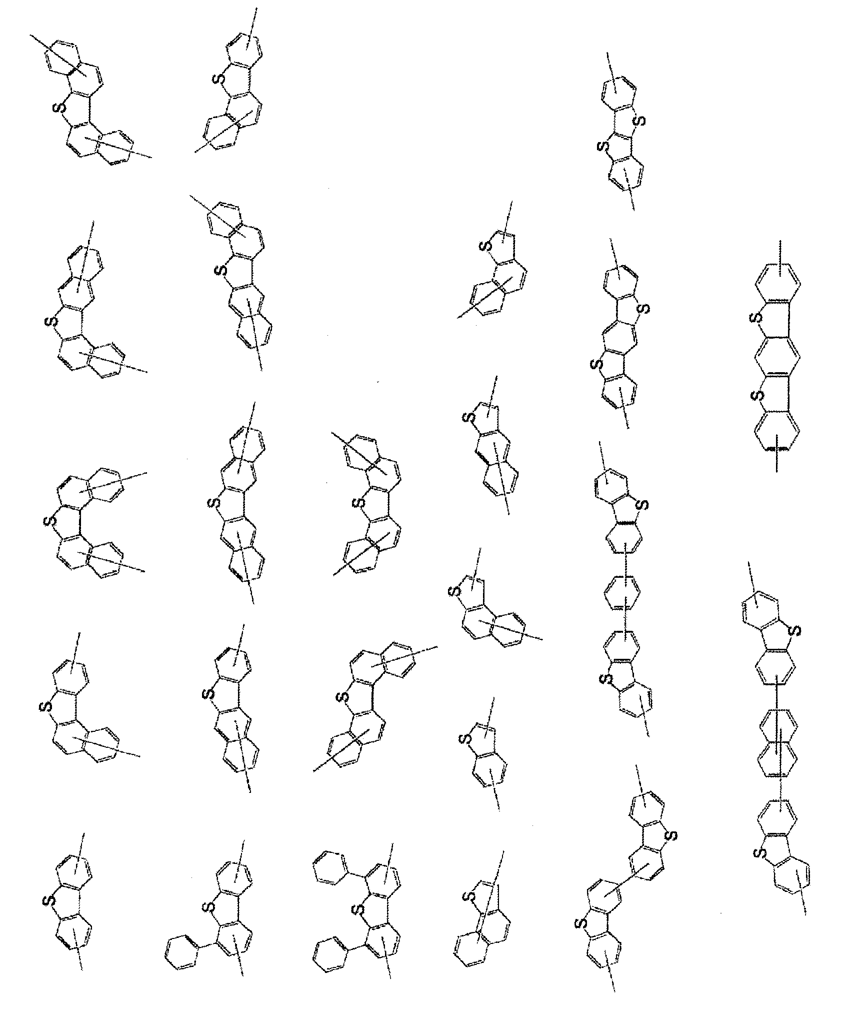

It is preferable that the benzothiophene derivative represented by general formula (1) is a derivative represented by any one of the following general formulae (2) and (3):

X-(B)ₘ-L-(C)ₙ-Y (2)

(A)ₕ-(X)ₖ-(B)ₘ-L (3)

In general formulae (2) and (3), X, A, B, C, Y, L, h, k, m and n are as defined above. Examples of the groups represented by X, A, B, C, Y and L include the groups described above as the examples of the corresponding groups.

Examples of the substituent in the groups represented by general formulae (1) to (7) include alkyl groups (such as methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodolsopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitro-ethyl group, 1,3-dinitroisopropyl group, 2,3-dinitro-t-butyl group, 1,2,3-trinitropropyl group, cyclopropyl group, cyclobutyl group, cyclopentyl group, cyclohexyl group, 4-methylcyclohexyl group, 1-adamantyl group, 2-adamantyl group, 1-norbornyl group and 2-norbornyl group), alkoxyl groups having 1 to 6 carbon atoms (such as ethoxyl group, methoxyl group, isopropoxyl group, n-propoxyl group, s-butoxyl group, t-butoxyl group, pentoxyl group, hexyloxyl group, cyclopentoxyl group and cyclohexyloxyl group), aryl groups having 5 to 40 ring atoms, amino groups substituted with an aryl group having 5 to 40 ring atoms, ester groups having an aryl group having 5 to 40 ring atoms, ester groups having an alkyl group having 1 to 6 carbon atoms, cyano group, nitro group and halogen atoms.

Specific examples of the benzothiophene derivative represented by general formula (1) of the present invention are shown in the following. However, the benzothiophene derivative is not limited to the compounds shown as the examples.

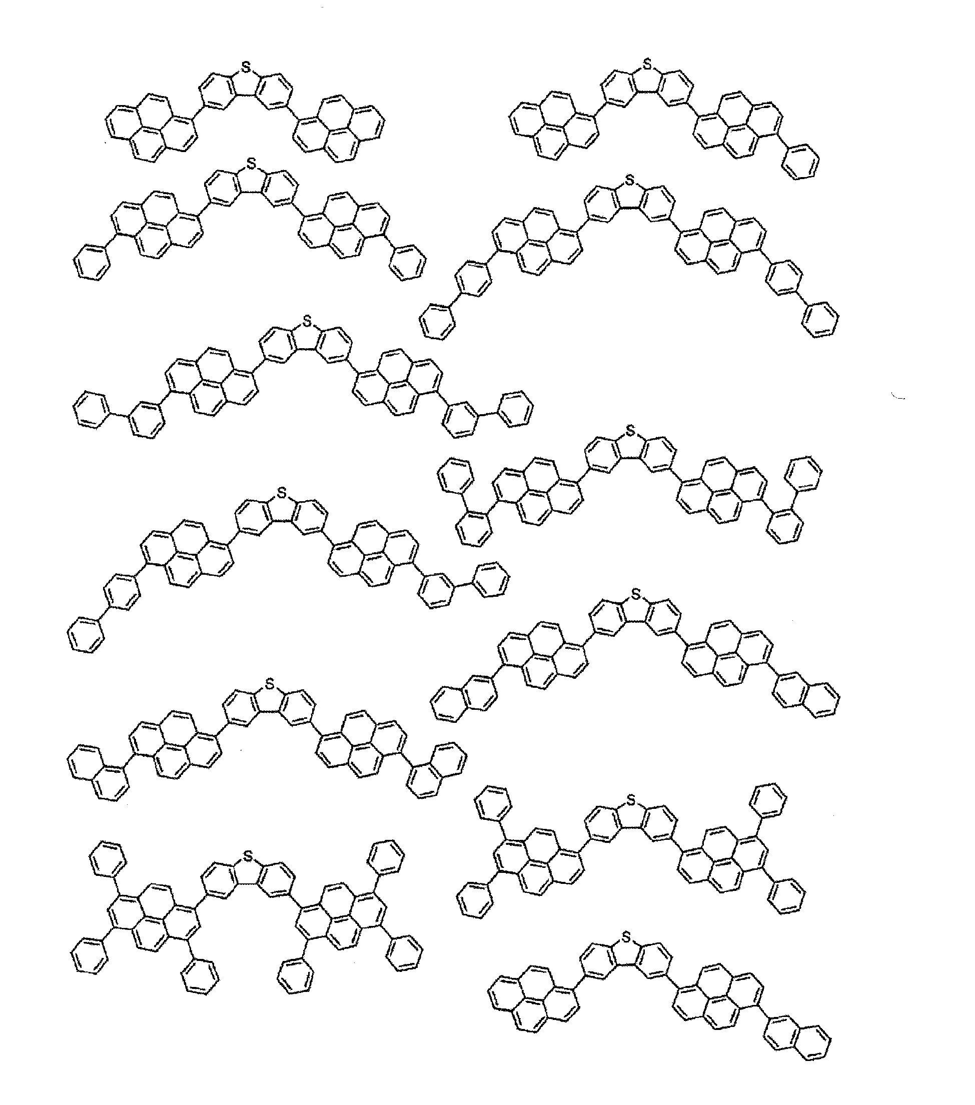

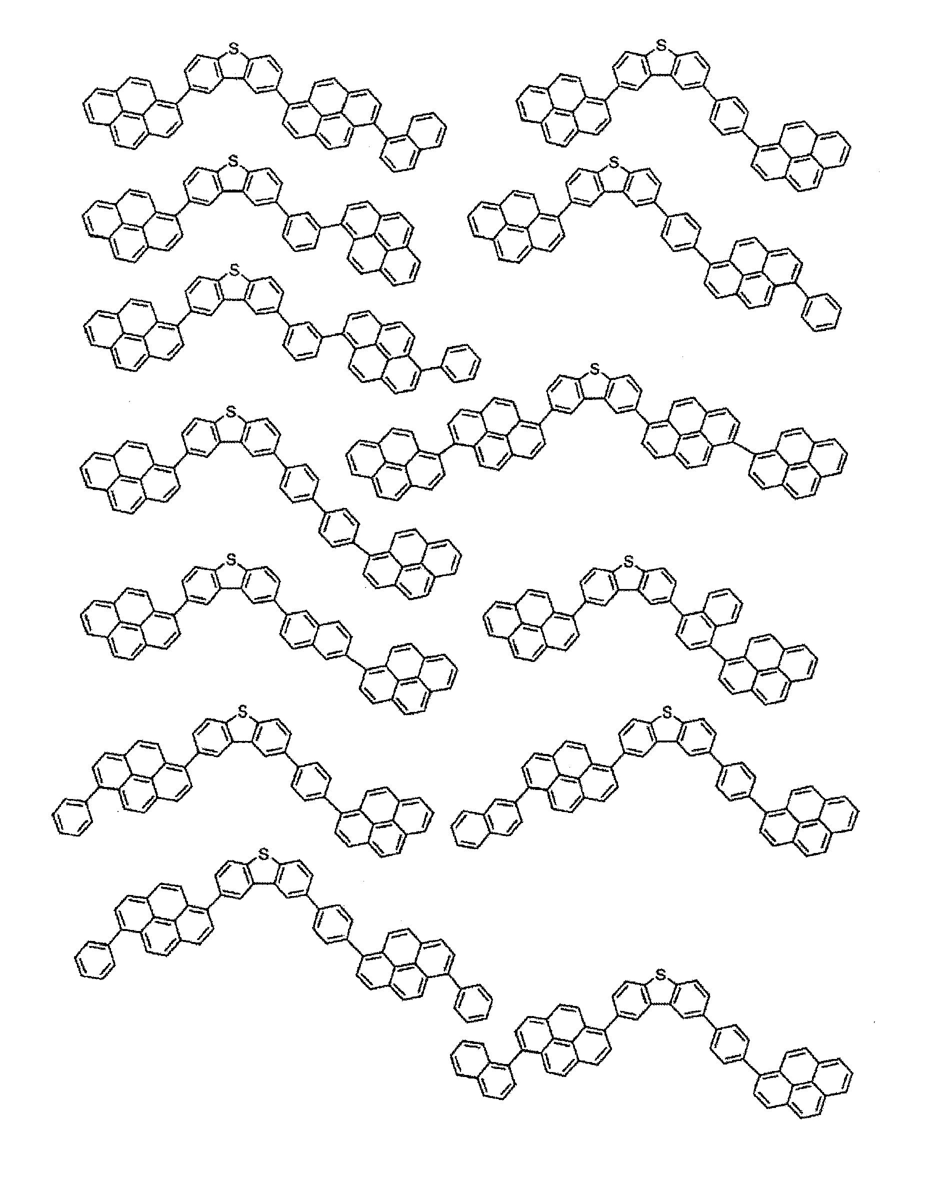

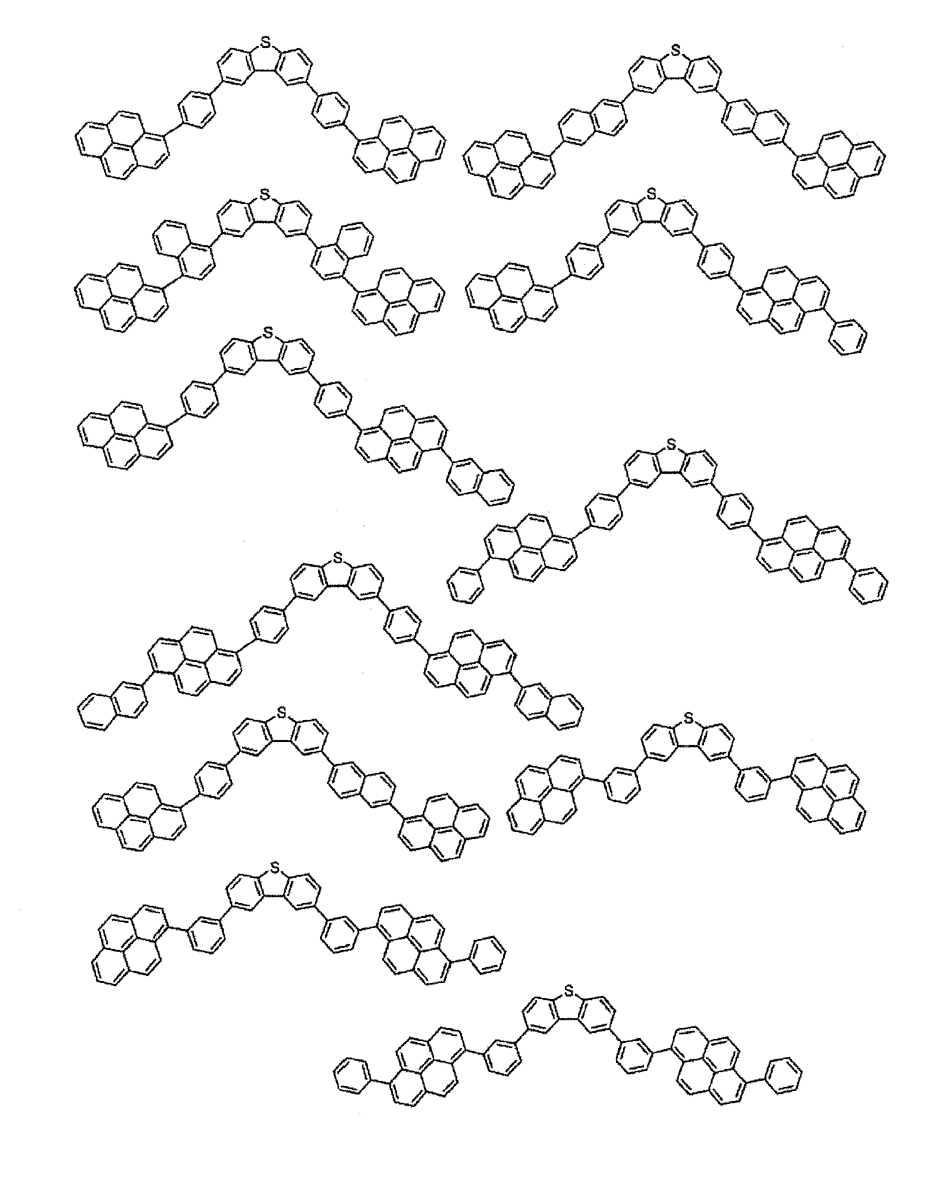

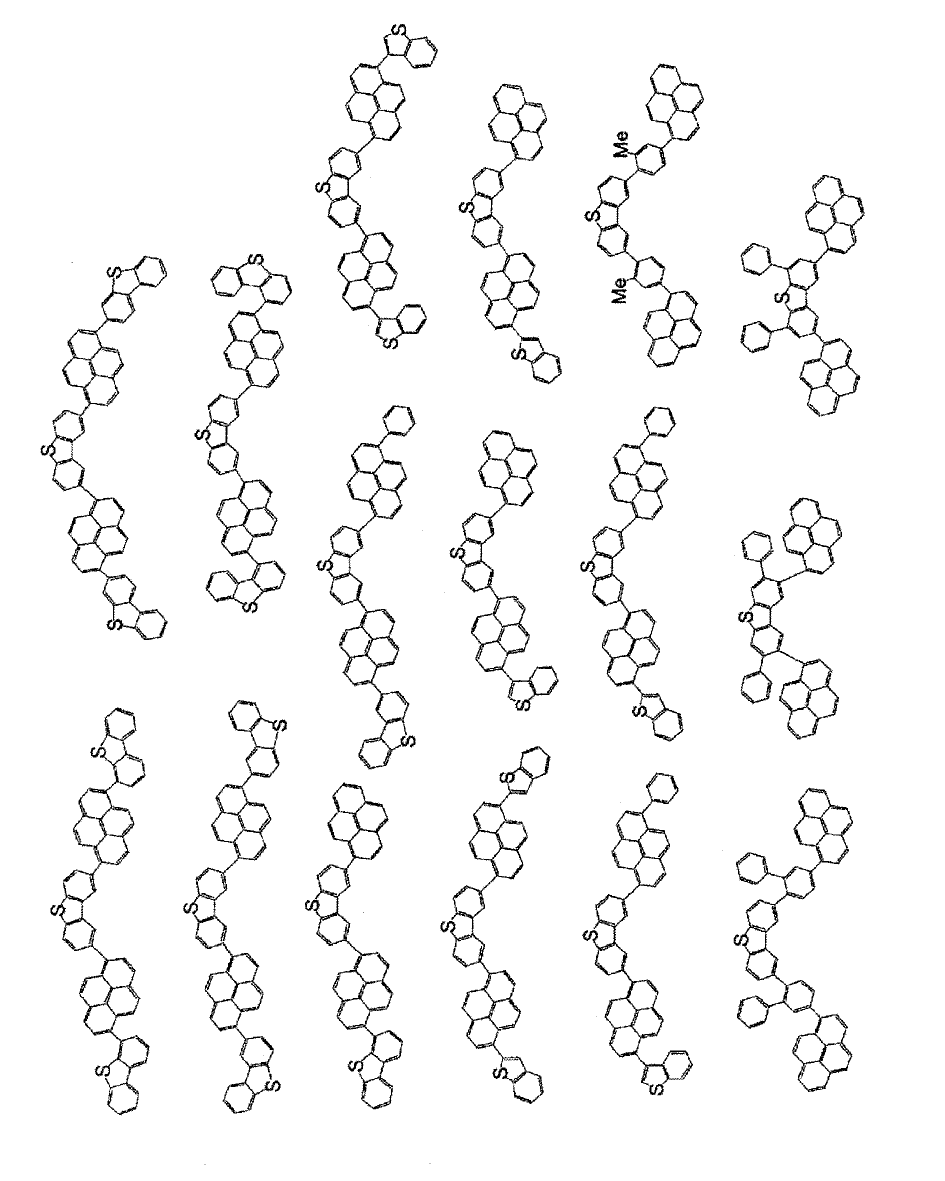

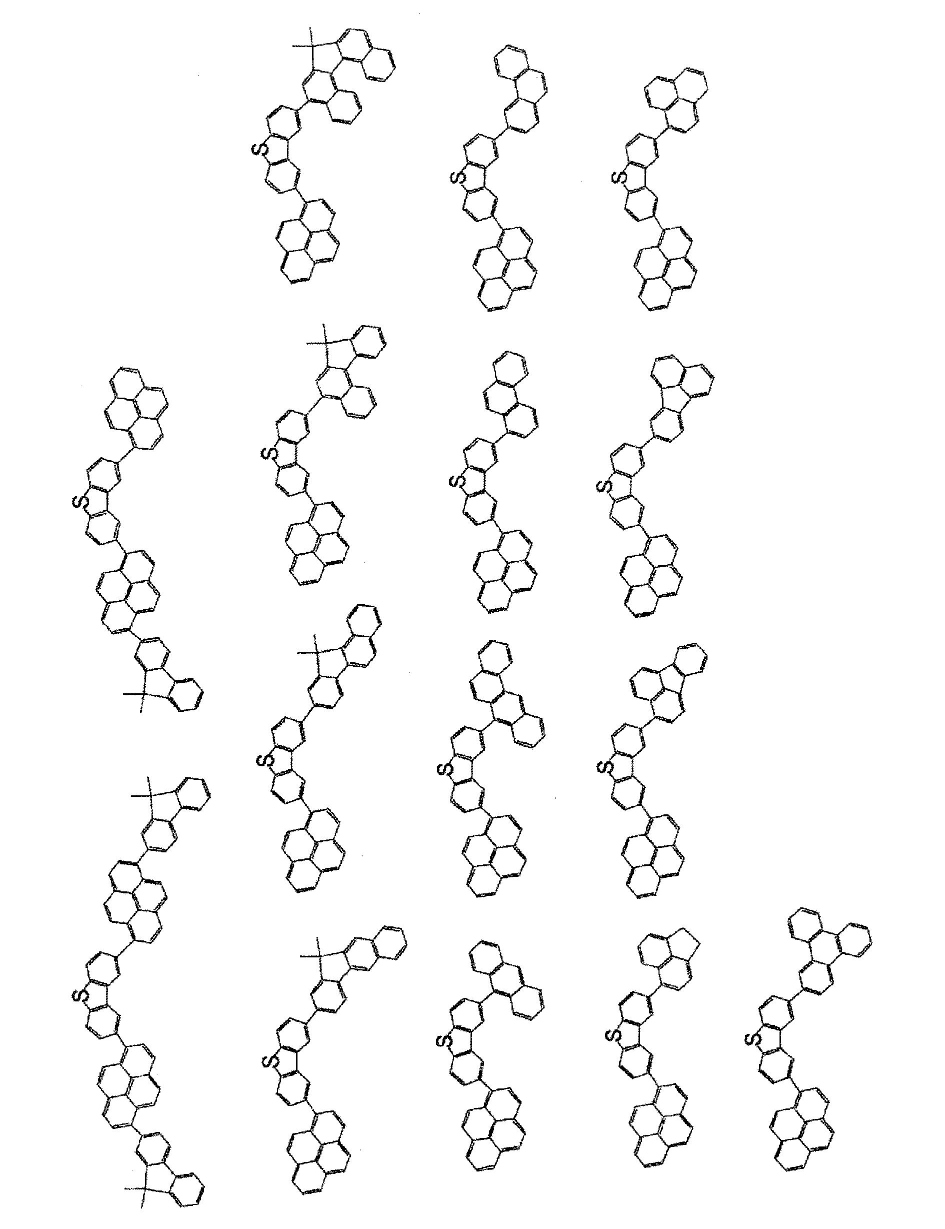

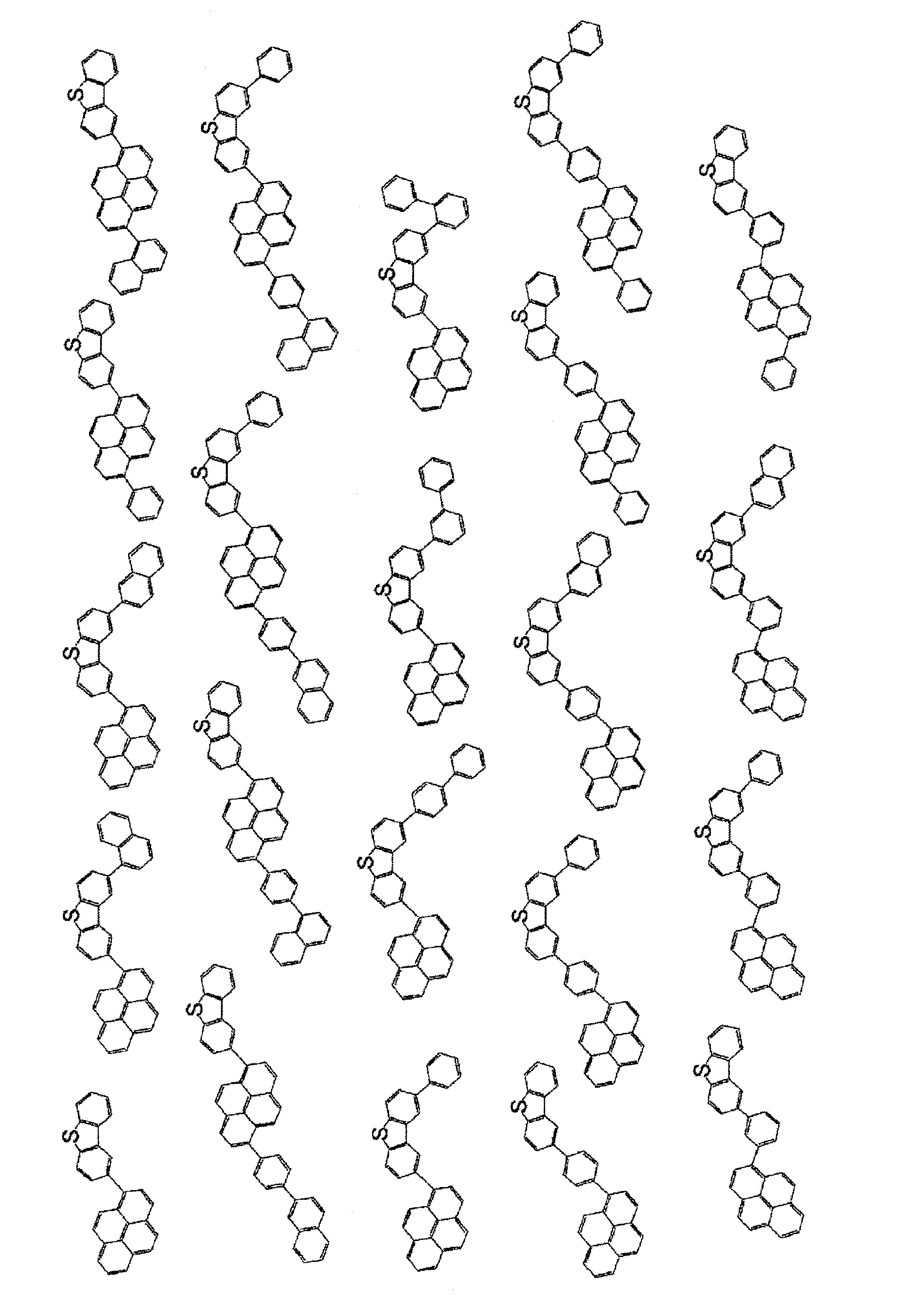

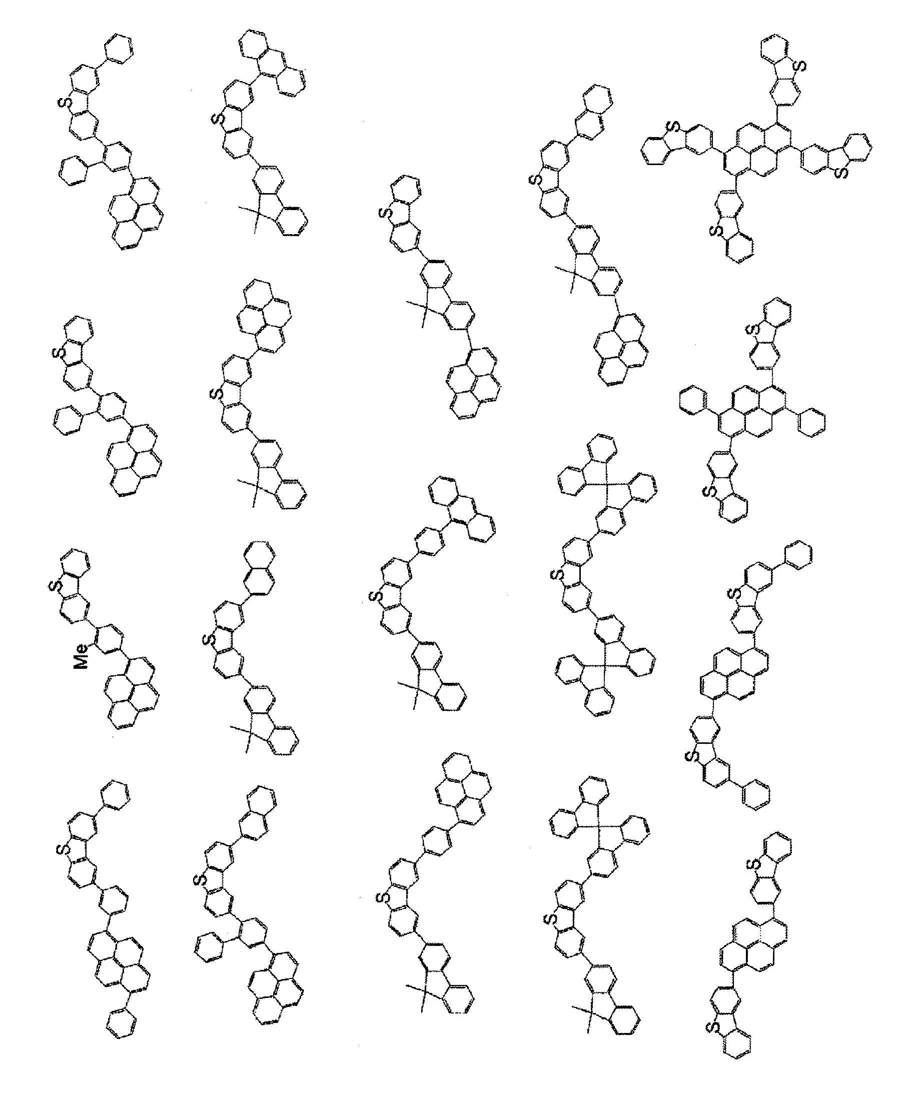

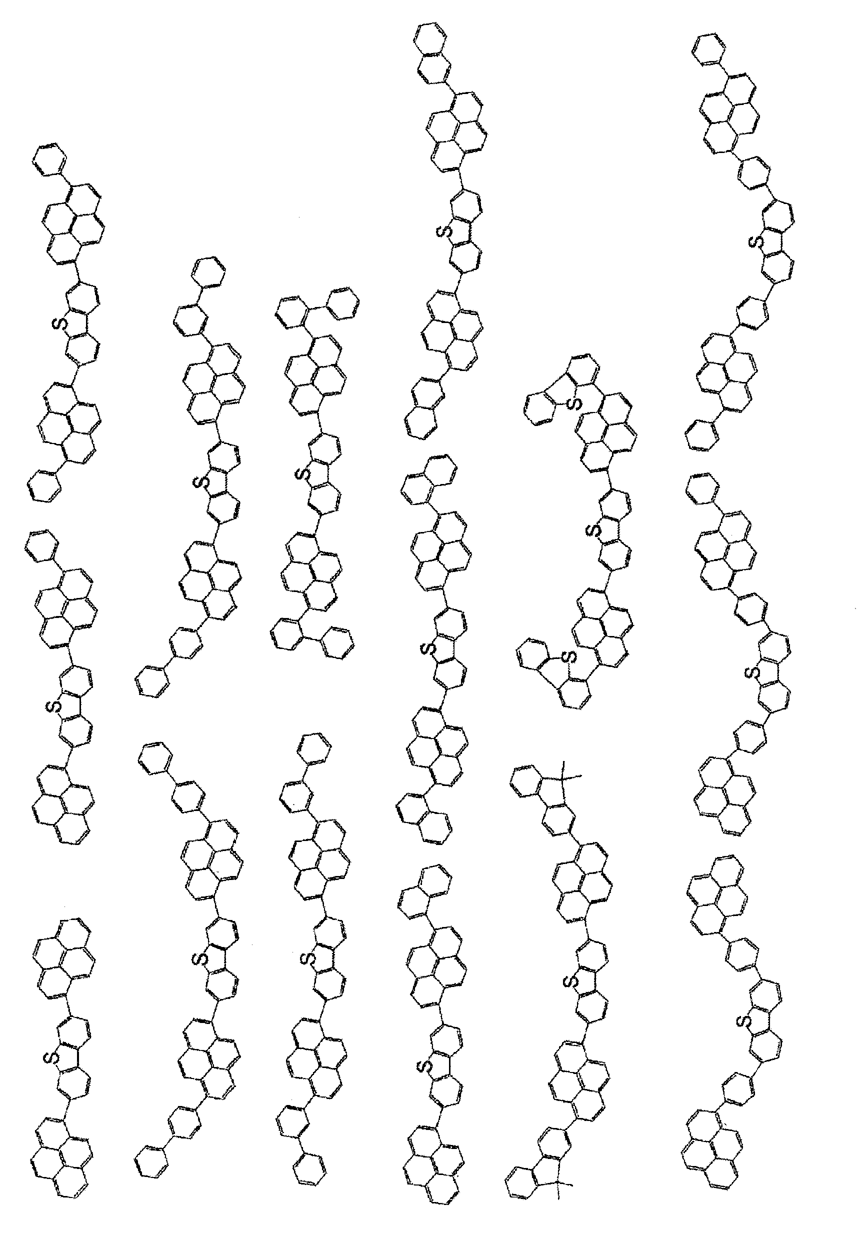

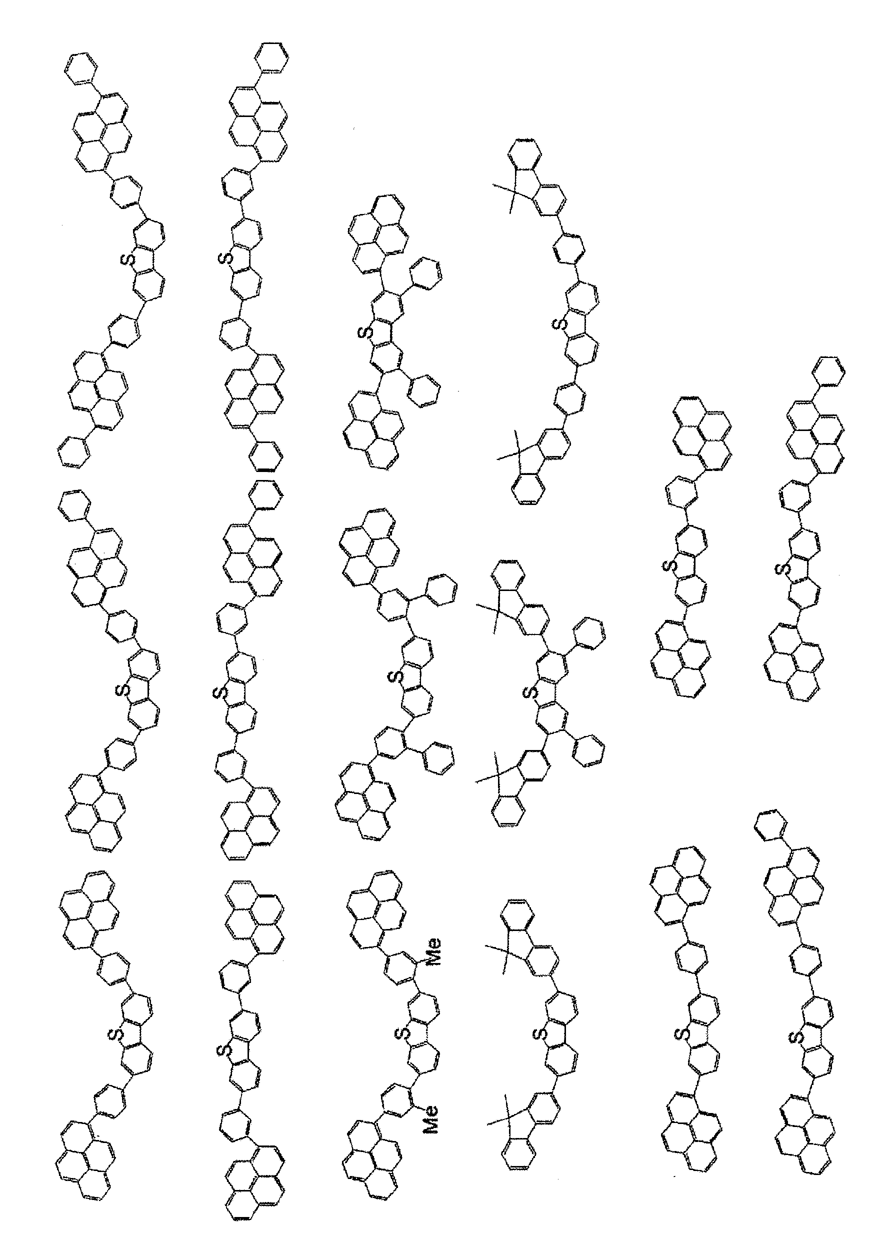

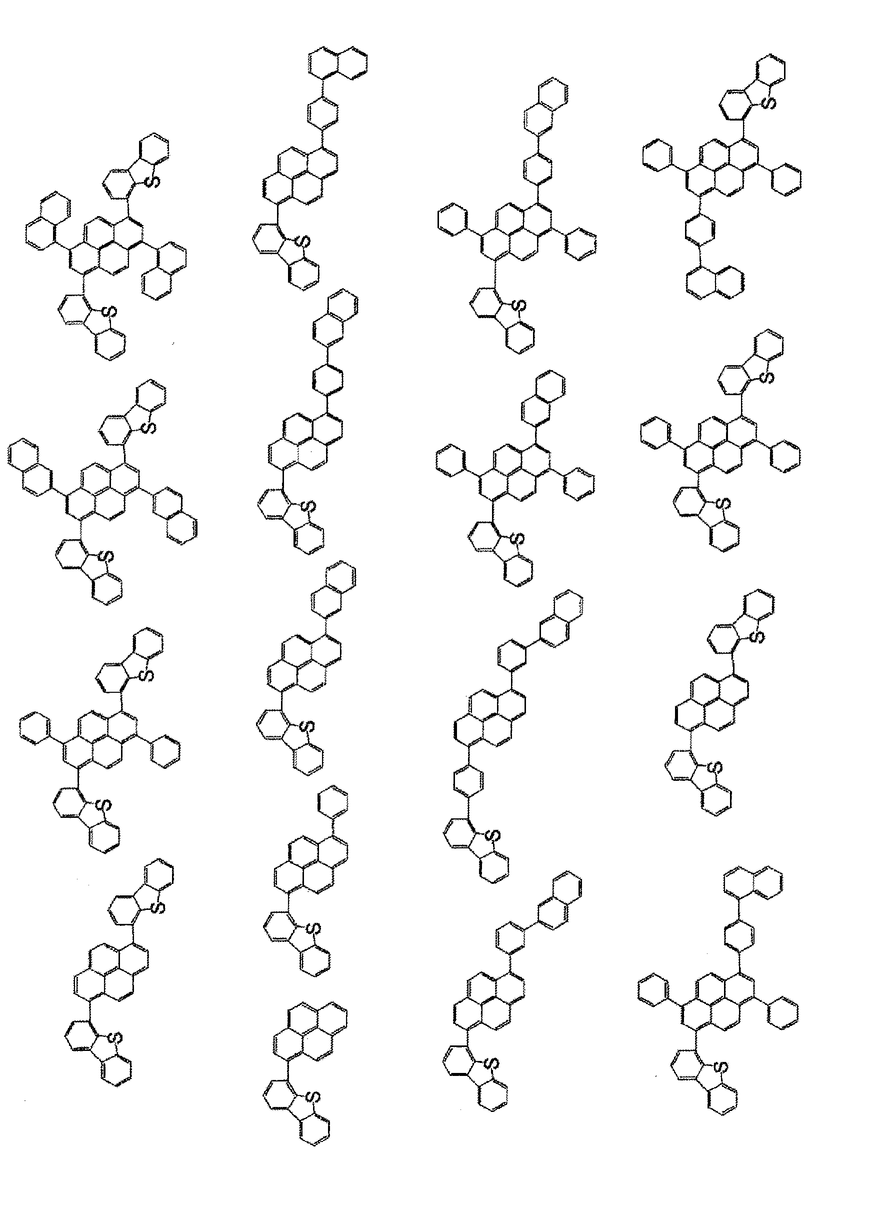

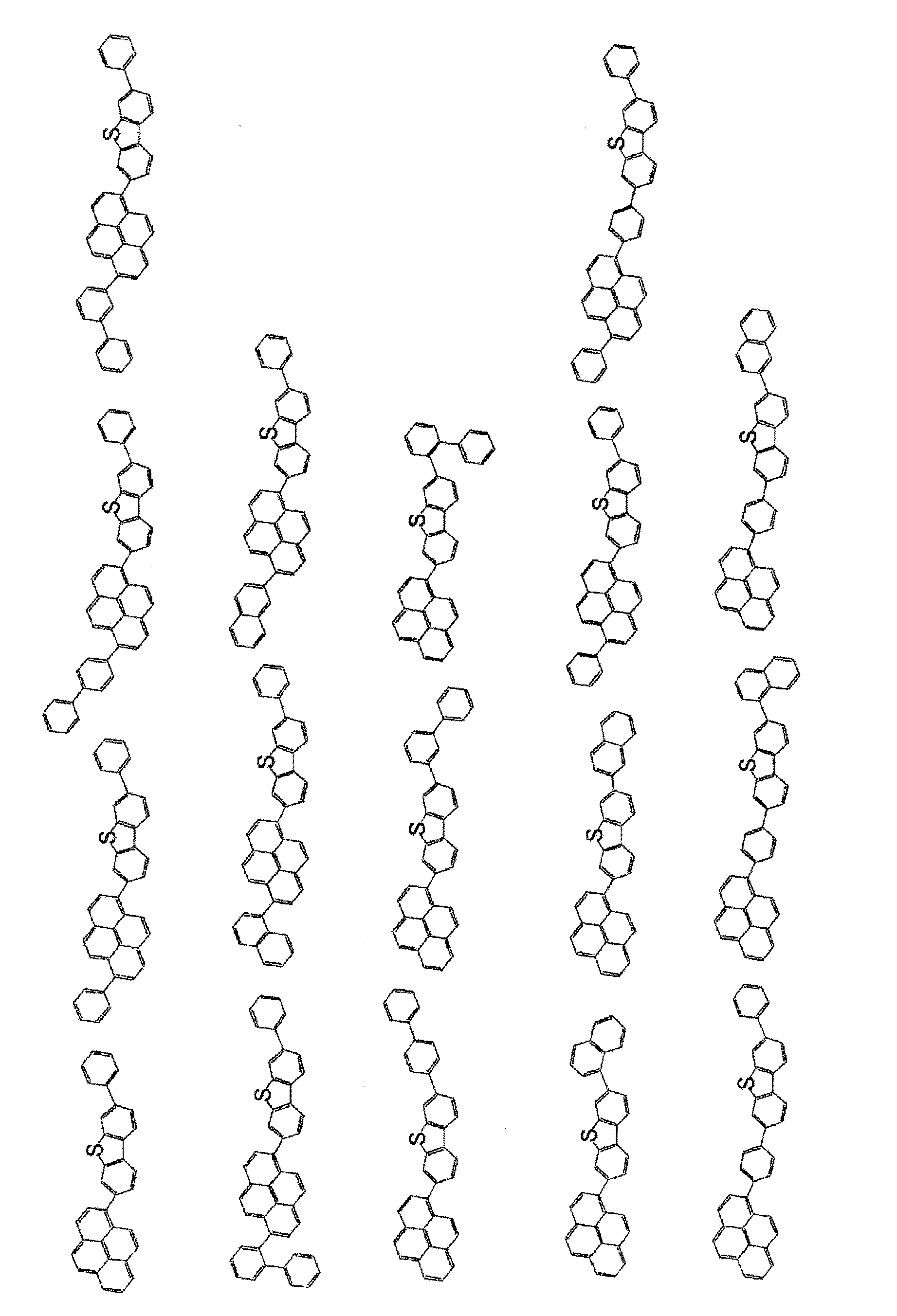

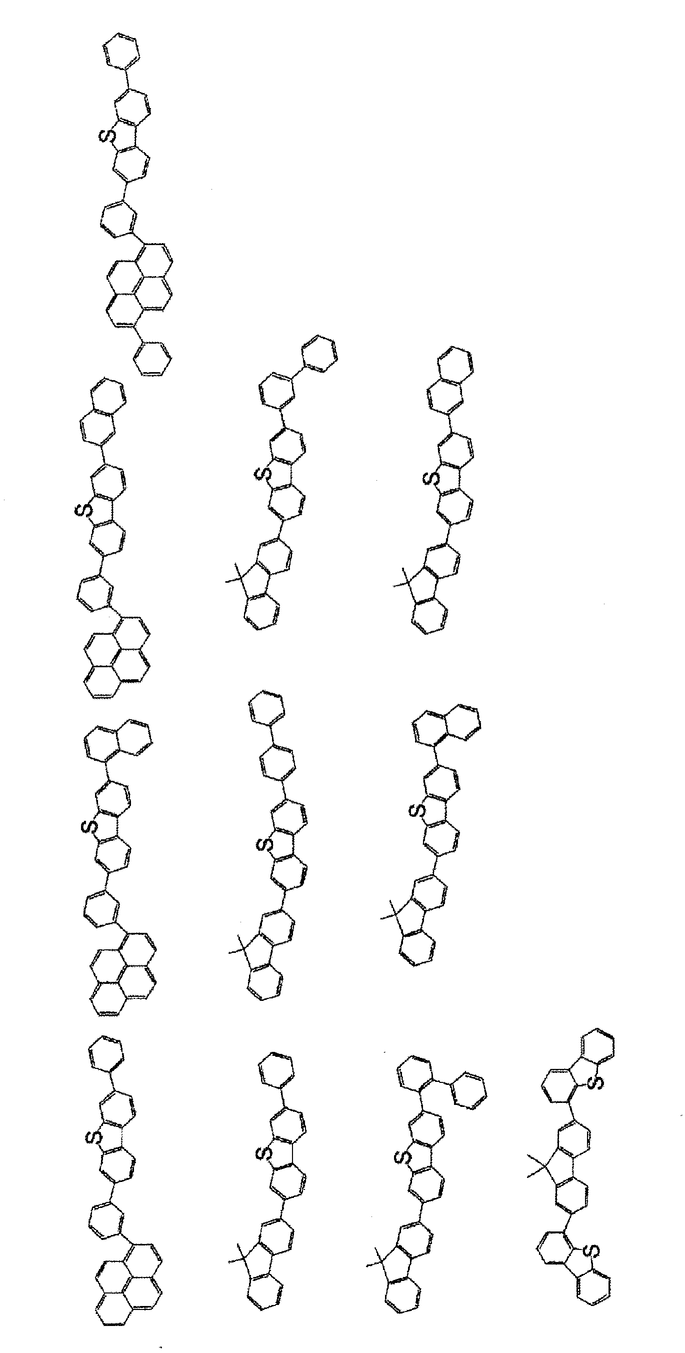

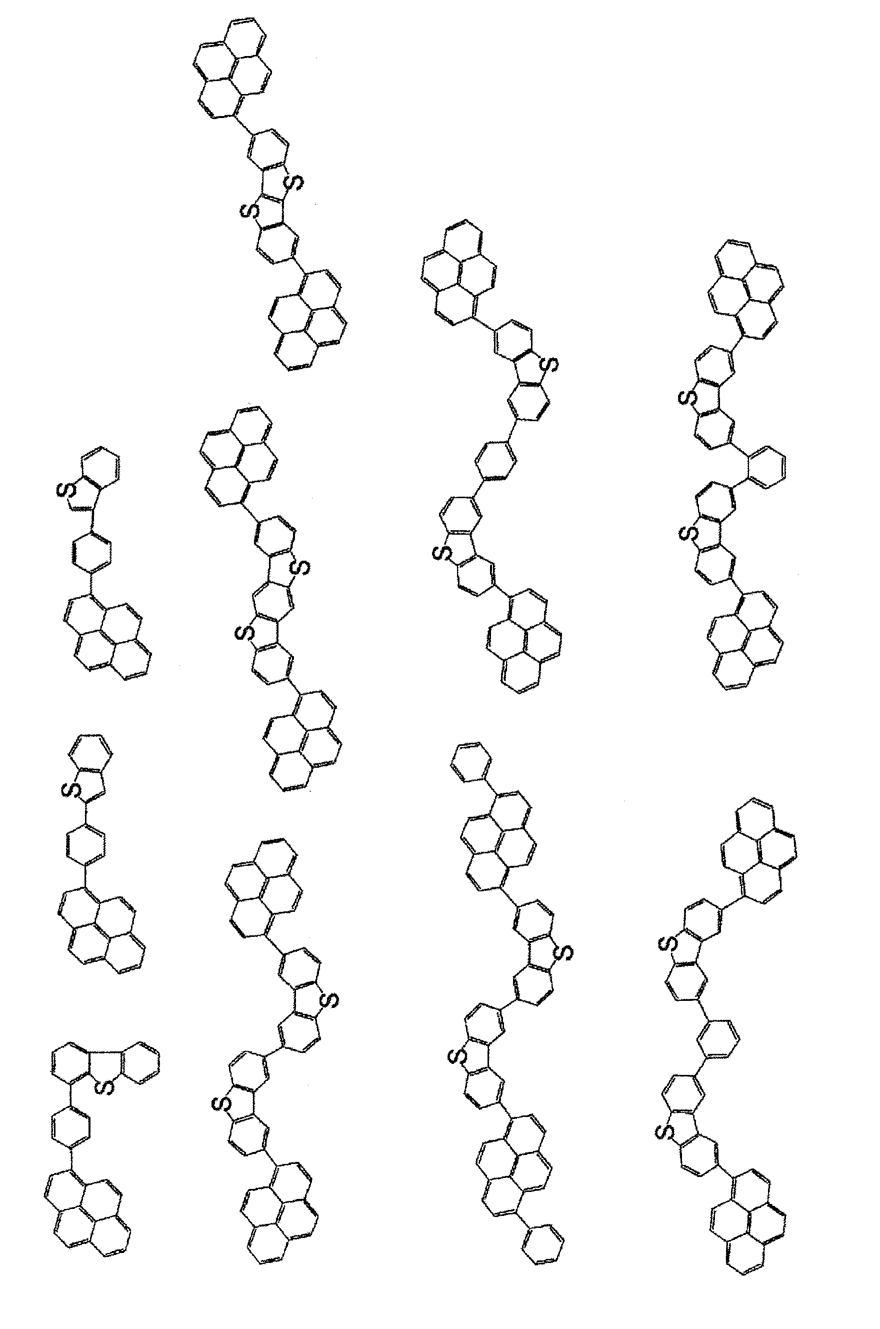

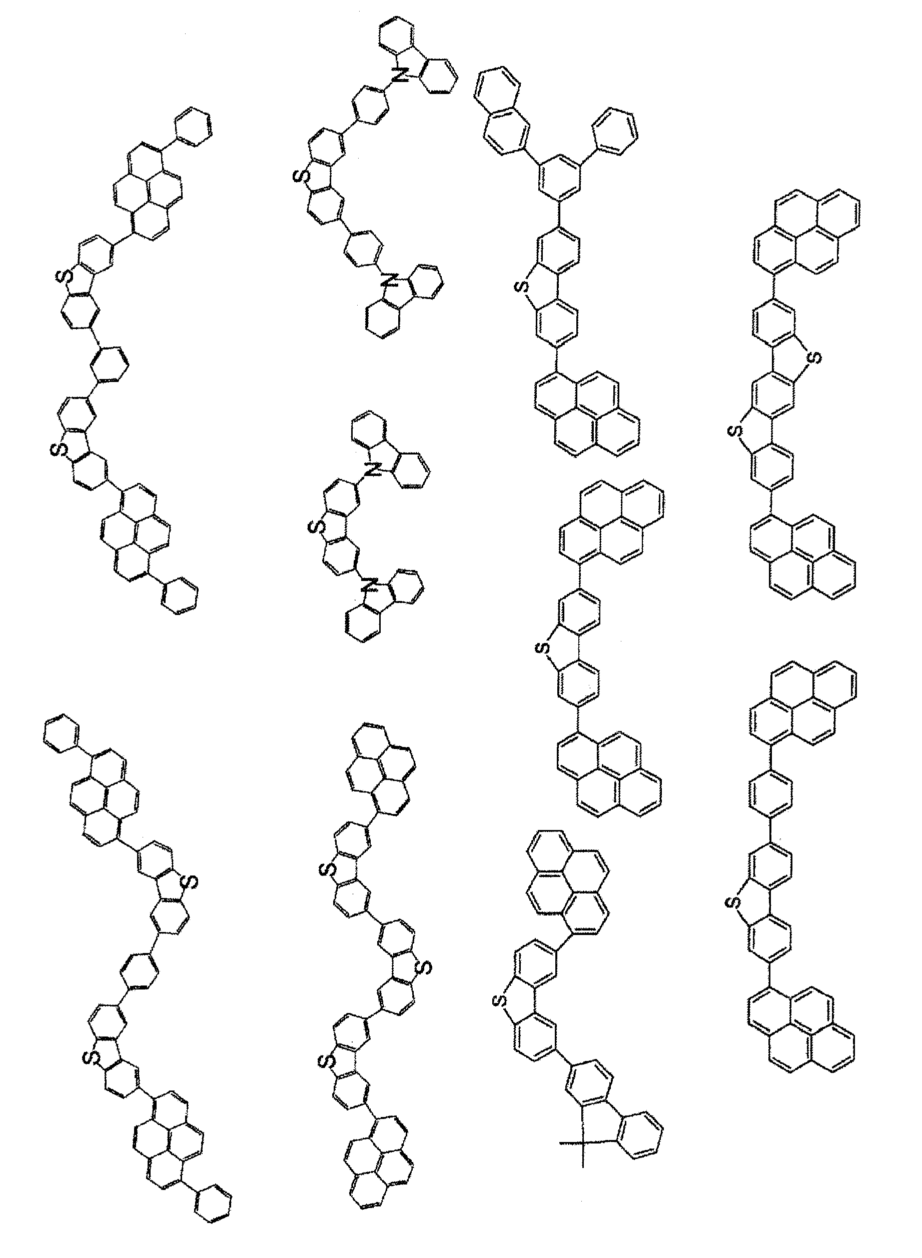

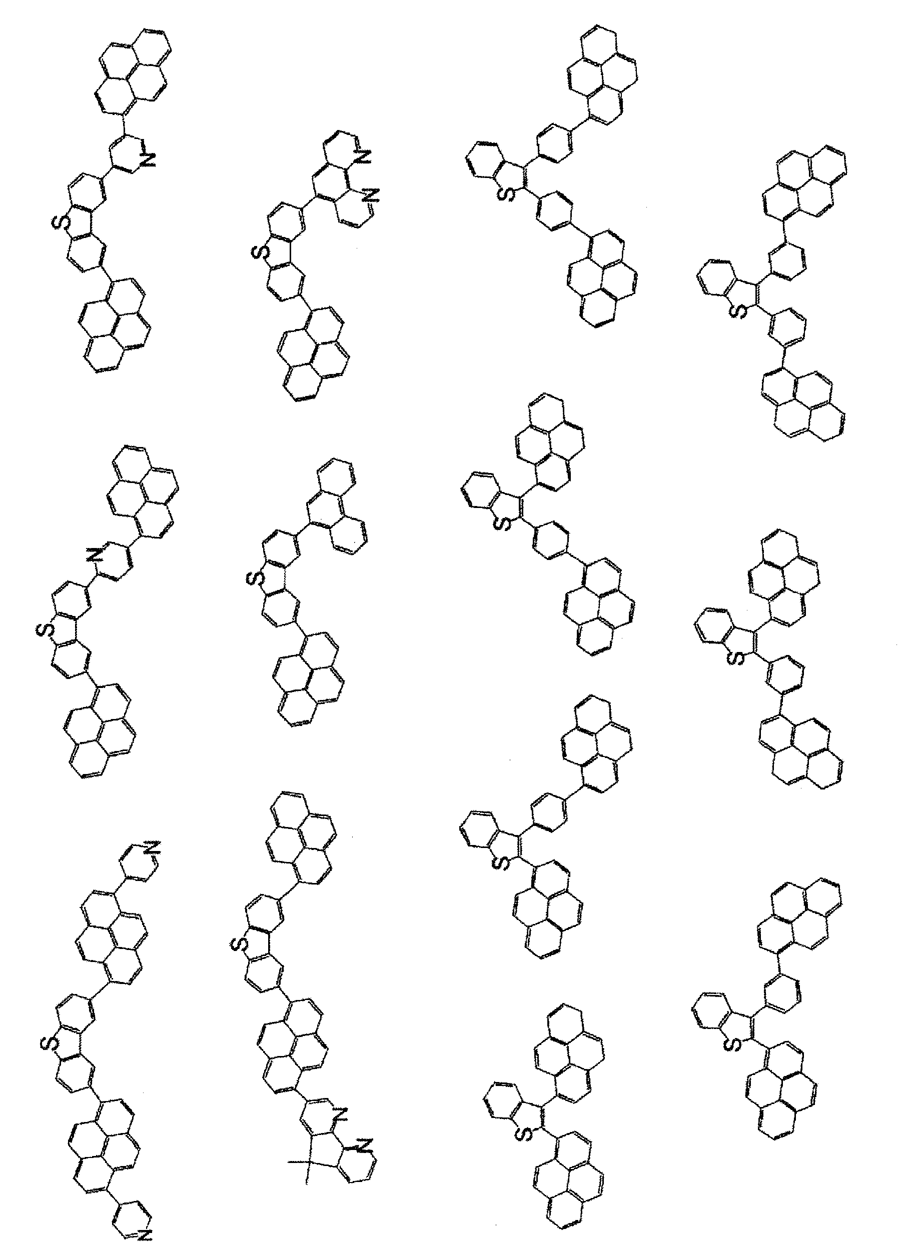

It is preferable that the benzothiophene derivative of the present invention is a material for organic EL devices and more preferably the host material for organic EL devices.

The organic EL device of the present invention is an electroluminescence device which comprises a cathode, an anode and an organic thin film layer which comprises at least one layer comprising at least a light emitting layer and is disposed between the cathode and the anode, wherein at least one layer in the organic thin film layer comprises the benzothiophene derivative represented by general formula (1) singly or as a component of a mixture.

It is preferable that the light emitting layer comprises the benzothiophene derivative represented by general formula (1) as the light emitting material. It is preferable that the light emitting layer comprises the benzothiophene derivative in an amount of 10 to 100% and more preferably 50 to 99%.

It is preferable that the content of sublimable impurities comprising halogen atoms in the light emitting layer is controlled to a specific value or smaller such as 100 ppm or smaller.

It is preferable that the light emitting layer in the organic EL device of the present invention further comprises an arylamine compound and/or a styrylamine compound.

As the styrylamine compound, compounds represented by the following general formula (A) are preferable.

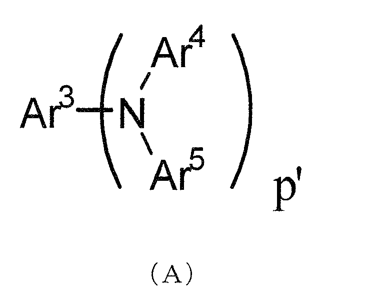

In general formula (A), Ar³ represents a group selected from phenyl group, biphenyl group, terphenyl group, stilbene group and distyrylaryl groups, Ar⁴ and Ar⁶ each represent hydrogen atom or an aromatic hydrocarbon group having 6 to 20 carbon atoms, the groups represented by Ar³, Ar⁴ and Ar⁵ may be substituted, and p' represents an integer of 1 to 4. It is preferable that at least one of the groups represented by Ar⁴ and Ar⁵ is substituted with styryl group.

Examples of the aromatic hydrocarbon group having 6 to 20 carbon atoms include phenyl group, naphthyl group, anthranyl group, phenanthryl group and terphenyl group.

As the arylamine compound, compounds represented by the following general formula (B) are preferable.

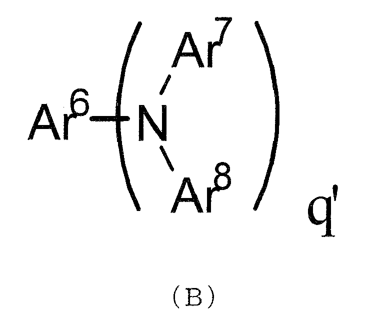

In general formula (B), Ar⁶ to Ar⁸ each represent an aryl group having 5 to 40 ring carbon atoms which may be substituted, and q' represents an integer of 1 to 4.

Examples of the aryl group having 5 to 40 ring carbon atoms include phenyl group, naphthyl group, anthranyl group, phenanthryl group, pyrenyl group, coronyl group, biphenyl group, terphenyl group, pyrrolyl group, furanyl group, thiophenyl group, benzothiophenyl group, oxadiazolyl group, diphenylanthranyl group, indolyl group, carbazolyl group, pyridyl group, benzoquinolyl group, fluoranthenyl group, acenaphthofluoranthenyl group, stilbene group, perylenyl group, chrysenyl group, pycenyl group, triphenylenyl group, rubicenyl group, benzoanthracenyl group, phenylanthranyl group, bisanthracenyl group, aryl groups represented by the following general formula (C) and an aryl group expressed by the following formula (D). Among these groups, naphthyl group, anthranyl group, chrysenyl group, pyrenyl group and the aryl group expressed by formula (D) are preferable. 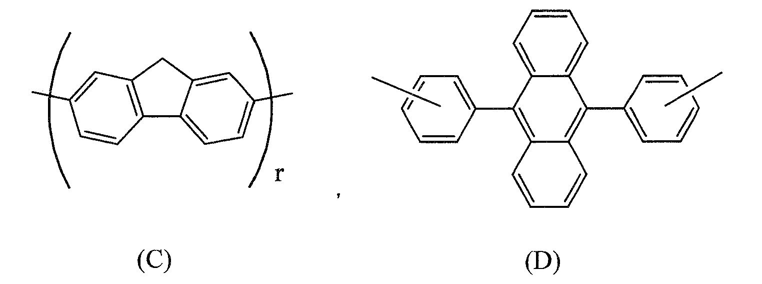 In general formula (C), r represents an integer of 1 to 3.

Preferable examples of the substituent to the aryl group include alkyl groups having 1 to 6 carbon atoms (such as ethyl group, methyl group, i-propyl group, n-propyl group, s-butyl group, t-butyl group, pentyl group, hexyl group, cyclopentyl group and cyclohexyl group), alkoxyl groups having 1 to 6 carbon atoms (such as ethoxyl group, methoxyl group, i-propoxyl group, n-propoxyl group, s-butoxyl group, t-butoxyl group, pentoxyl group, hexyloxyl group, cyclopentoxyl group and cyclohexyloxyl group), aryl groups having 5 to 40 ring carbon atoms, amino groups substituted with an aryl group having 5 to 40 ring carbon atoms, ester groups having an aryl group having 5 to 40 ring carbon atoms, ester groups having an alkyl group having 1 to 6 carbon atoms, cyano group, nitro group and halogen atoms.

The light emitting layer may comprise a fluorescent or phosphorescent compound in combination with the compound represented by general formula (1).

Examples of the fluorescent dopant include amine-based compounds, chelate complex compounds such as complex compounds of tris(8-quinolinolato)aluminum, coumarine derivatives, tetraphenylbutadiene derivatives, bisstyrylarylene derivatives and oxadiazole derivative. It is preferable that a compound is selected and used in accordance with the required color of the emitted light.

As the phosphorescent dopant, metal complexes having at least one metal selected from Ir, Ru, Pd, Pt, Os and Re are preferable. It is preferable that the ligand has at least one skeleton structure selected from the phenylpyridine skeleton structure, the bipyridyl skeleton structure and the phenanthroline skeleton structure. Examples of the metal complex compound described above include tris(2-phenylpyridine)-iridium, tris(2-phenylpyridine)ruthenium, tris(2-phenylpyridine)-palladium, bis(2-phenylpyridine)platinum, tris(2-phenylpyridine)osmium, tris(2-phenylpyridine)rhenium, octaethylplatinumporphyrin, octaphenylplatinumporphyrin, octaethylpalladiumporphyrin and octaphenylpalladiumporphyrin. However, the metal complex compound is not limited to the compounds described above as the examples. It is preferable that the metal complex compound is selected in accordance with the required color of emitted light, the properties of the device and the relation with the host material.

The construction of the organic EL device of the present invention will be described in the following.

The organic EL device of the present invention is a device prepared by forming at least one organic thin film layer between an anode and a cathode. When the organic thin film layer comprises a single layer, a light emitting layer is disposed between the anode and the cathode. The light emitting layer comprises a light emitting material and may further comprise a hole injecting material or an electron injecting material for transporting holes injected from the anode or electrons injected from the cathode, respectively, to the light emitting material. It is preferable that the light emitting material has a great quantum efficiency of fluorescence, a combination of excellent ability of transporting holes and excellent ability of transporting electrons and can form a uniform film.

Examples of the construction of the device of the multiple layer type include constructions obtained by laminating a plurality of layers such as (an anode / a hole injecting layer / a light emitting layer / a cathode), (an anode / a light emitting layer / an electron injecting layer / a cathode), (an anode / a hole injecting layer / a light emitting layer / an electron injecting layer / a cathode) and (an anode / a hole injecting layer / a hole transporting layer / a light emitting layer / an electron injecting layer / a cathode).

In the light emitting layer, where necessary, conventional light emitting materials, doping materials, hole injecting materials and electron injecting materials may be used in combination with the compound represented by general formula (1) of the present invention.

As the doping material, conventional fluorescent materials can be used, and any of complexes of heavy metals such as iridium having the phosphorescent property may also be used. Decreases in the luminance and the life of the organic EL device due to quenching can be prevented by using a multi-layer structure for the organic thin film layer. Where necessary, the light emitting materials, the doping materials, the hole injecting materials and the electron injecting materials may be used in combination. By the use of the doping material, the luminance of the emitted light and the efficiency of the light emission can be increased, and red light or white light can be emitted.

The hole injecting layer, the light emitting layer and the electron injecting layer may each have a structure having two or more layers. In this case, in the hole injecting layer, the layer into which holes are injected from the electrode is called the hole injecting layer, and the layer which receives the holes from the hole injecting layer and transports the holes to the light emitting layer is called the hole transporting layer. Similarly, in the electron injecting layer, the layer into which electrons are injected from the electrode is called the electron injecting layer, and the layer which receives the electrons from the electron injecting layer and transports the electrons to the light emitting layer is called the electron transporting layer. These layers are selected and used in accordance with various factors such as the energy level of the material, the heat resistance of the material and the adhesion of the material with the organic layer or the metal electrode.

Examples of the light emitting material and the host material used in the organic thin film layer in combination with the compound represented by general formula (1) include anthracene, naphthalene, phenanthrene, pyrene, tetracene, coronene, chrysene, fluoresceine, perylene, phthaloperylene, naphthaloperylene, perynone, phthaloperynone, naphthaloperynone, diphenylbutadiene, tetraphenylbutadiene, coumarine, oxadiazole, aldazine, bisbenzoxazoline, bistyryl, pyrazine, cyclopentadiene, metal complexes of quinoline, metal complexes of aminoquinoline, metal complexes of benzoquinoline, imine, diphenylethylene, vinylanthracene, diaminocarbazole, pyrane, thiopyrane, polymethine, melocyanine, oxinoid compounds chelated with imidazole, quinacridone, rubrene, stilbene-based derivatives and fluorescent coloring agents. However, the light emitting material and the host material are not limited to the above compounds.

As the hole injecting and transporting material, compounds which have the ability of transporting holes, exhibit the excellent effect of injection of holes from the anode and the excellent effect of injection of holes to the light emitting layer or the light emitting material, prevent transfer of excimers formed in the light emitting layer to the electron injecting layer or the electron injecting material and have excellent ability of forming a thin film are preferable. Examples of the hole injecting material include phthalocyanine derivatives, naphthalocyanine derivatives, porphyrin derivatives, oxazoles, oxadiazoles, triazoles, imidazoles, imidazolones, imidazolethiones, pyrazolines, pyrazolones, tetrahydroimidazoles, oxazoles, oxadiazoles, hydrazones, acylhydrazones, polyarylalkanes, stilbenes, butadienes, triphenylamines of the benzidine type, triphenylamines of the styrylamine type, triphenylamines of the diamine type, derivatives of these compounds and macromolecular materials such as polyvinylcarbazole, polysilanes and electrically conductive macromolecules. However, the hole injecting material is not limited to the compounds described above.

Among the hole injecting and transporting materials which can be used in the organic EL device of the present invention, aromatic tertiary amine derivatives and phthalocyanine derivatives are more effective hole injecting and transporting materials.

Examples of the aromatic tertiary amine derivative include triphenylamine, tritolylamine, tolyldiphenylamine, N,N'-diphenyl-N,N'-(3-methylphenyl)-1,1'-biphenyl-4,4'-diamine, N,N,N',N'-(4-methylphenyl) 1,1'-phenyl-4,4'-diamine, N,N,N',N'-(4-methylphenyl)-1,1'-biphenyl-4,4'-diamine, N,N'-diphenyl-N,N'-dinaphthyl-1,1'-biphenyl-4,4'-diamine, N,N'-(methylphenyl)-N,N'-(4-n-butylphenyl)phenanthrene-9,10-diamine, N,N-bis(4-di-4-tolylaminophenyl)-4-phenylcyclohexane and oligomer and polymers having the skeleton structure of the above aromatic tertiary amines. However, the aromatic tertiary amine derivative is not limited to the compounds described above.

Examples of the phthalocyanine (Pc) derivative include phthalocyanine derivatives and naphthalocyanine derivatives such as H₂Pc, CuPc, CoPc, NiPc, ZnPc, PdPc, FePc, MnPc, ClAlPc, CIGaPc, ClInPc, CISnPc, Cl₂SiPc, (HO)AlPc, (HO)GaPc, VOPc, TiOPc, MoOPc and GaPc-O-GaPc. However, the phthalocyanine derivative is not limited to the compounds described above.

As the electron injecting and transporting material, compounds which have the ability of transporting electrons, exhibit the effect of injection of electrons from the cathode and the excellent effect of injection of electrons into the light emitting layer or the light emitting material, prevent transfer of excimers formed in the light emitting layer into the hole injecting layer and have excellent ability of forming a thin film, are preferable. Examples of the electron injecting material include fluorenone, anthraquinodimethane, diphenoquinones, thiopyrane dioxides, oxazoles, oxadiazoles, triazoles, imidazoles, perylenetetracarboxylic acid, fluorenylidenemethane, anthraquinodimethane, anthrone and derivatives of these compounds. However, the electron injecting and transporting material is not limited to the compounds described above.

The charge injecting property can be improved by adding an electron-accepting substance to the hole injecting and transporting material and by adding an electron-donating substance to the electron injecting and transporting material.

In the organic EL device of the present invention, more effective electron injecting and transporting materials among electron injecting and transporting materials which can be used are metal complex compounds and five-membered cyclic derivatives having nitrogen atom.

Examples of the metal complex compound include 8-hydroxy-quinolinatolithium, bis(8-hydroxyquinolinato)zinc, bis(8-hydroxy-quinolinato)copper, bis(8-hydroxyquinolinato)manganese, tris(8-hydroxy-quinolinato)aluminum, tris(2-methyl-8-hydroxyquinolinato)aluminum, tris(8-hydroxyquinolinato)gallium, bis(10-hydroxybenzo[h]quinolinato)-beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum and bis(2-methyl-8-quinolinato)(2-naphtholato)gallium. However, the metal complex compound is not limited to the compounds described above.

As the five-membered cyclic derivative having nitrogen atom, for example, derivatives of oxazole, thiazole, oxadiazole, thiadiazole and triazole are preferable. Examples of the five-membered cyclic derivative having nitrogen atom include 2,5-bis(1-phenyl)-1,3,4-oxazole, dimethylPOPOP, 2,5-bis(1-phenyl)-1,3,4-thiazole, 2,5-bis(1-phenyl)-1,3,4-oxadiazole, 2-(4'-tert-butylphenyl)-5-(4"-biphenyl)-1,3,4-oxadiazole, 2,5-bis(1-naphthyl)-1,3,4-oxadiazole, 1,4-bis[2-(5-phenyloxadiazolyl)]benzene, 1,4-bis[2-(5-phenyloxadiazolyl)-4-tert-butylbenzene], 2-(4'-tert-butylphenyl)-5-(4"-biphenyl)-1,3,4-thiadiazole, 2,5-bis(1-naphthyl)-1,3,4-thiazole, 1,4-bis-[2-(5-phenylthiadiazolyl)]benzene, 2-(4'-t-butylphenyl)-5-(4"-biphenyl)-1,3,4-triazole, 2,5-bis(1-naphthyl)-1,3,4-triazole and 1,4-bis-[2-(5-phenyltriazolyl)]benzene. However, the five-membered cyclic derivative having nitrogen atom is not limited to the compounds described above.

In the organic EL device of the present invention, the light emitting layer may further comprise at least one of light emitting materials, doping materials, hole injecting and transporting materials and electron injecting and transporting materials in the same layer in combination with the compound represented by general formula (1). A protective layer may be formed on the surface of the device, or the entire device may be protected with a silicone oil or a resin so that stability of the organic EL device obtained in accordance with the present invention with respect to the temperature, the moisture and the atmosphere is improved.

As the electrically conductive material used for the anode in the organic EL device of the present invention, a material having a work function greater than 4 eV is suitable. Carbon, aluminum, vanadium, iron, cobalt, nickel, tungsten, silver, gold, platinum, palladium, alloys of these metals, metal oxides such as tin oxide and indium oxide used for ITO substrates and NESA substrates and organic electrically conductive resins such as polythiophene and polypyrrol can be used.

As the electrically conductive material used for the cathode in the organic EL device of the present invention, a material having a work function smaller than 4 eV is suitable. Magnesium, calcium, tin, lead, titanium, yttrium, lithium, ruthenium, manganese, aluminum and alloys of these metals can be used, but the material is not limited to these materials. Typical examples of the alloy include magnesium/silver, magnesium/indium and lithium/aluminum, but the alloy is not limited to these alloys. The ratio of the amounts of the components in the alloy is controlled by the temperature of the sources of vapor deposition, the atmosphere and the degree of vacuum and adjusted to a suitable value.

The anode and the cathode may have a structure having two or more layers, where necessary. In the organic EL device of the present invention, it is desirable that at least one face of the device is sufficiently transparent in the wavelength range of the light emitted from the device so that the emitted light is efficiently obtained.

It is desirable that the substrate used in the organic EL device of the present invention is transparent. The transparent electrode is formed in accordance with a process such as the vapor deposition process or the sputtering process using the above electrically conductive material in a manner such that the prescribed transparency is assured. It is preferable that the electrode on the light emitting face has a transmittance of light of 10% or greater. The substrate is not particularly limited as long as the substrate has sufficient mechanical and thermal strength and transparency. Examples of the substrate include glass substrates and transparent resin films. Examples of the transparent resin film include films of polyethylene, ethylene-vinyl acetate copolymers, ethylene-vinyl alcohol copolymers, polypropylene, polystyrene, polymethyl methacrylate, polyvinyl chloride, polyvinyl alcohol, polyvinyl butyral, nylons, polyether ether ketones, polysulfones, polyether sulfones, tetrafluoroethylene-perfluoroalkyl vinyl ether copolymers, polyvinyl fluoride, tetrafluoroethylene-ethylene copolymers, tetrafluoroethylene-hexafluoropropylene copolymers, polychlorotrifluoro-ethylene, polyvinylidene fluoride, polyesters, polycarbonates, polyurethanes, polyimides, polyether imides and polypropylene.

To the formation of the layers in the organic EL device of the present invention, any of dry film forming processes such as the vacuum vapor deposition process, the sputtering process, the plasma process and the ion plating process and wet film forming processes such as the spin coating process, the dipping process and the flow coating process can be applied. It is necessary that the thickness of the film is set at a suitable value although the thickness of the layer is not particularly limited. When the thickness is excessively great, a great voltage must be applied to obtain the prescribed output, and the efficiency decreases. When the thickness is excessively small, defects such as pin holes are formed, and the sufficient luminance of the emitted light cannot be obtained under application of an electric field. A thickness in the range of 5 nm to 10 µm is suitable, and a thickness in the range of 10 nm to 0.2 µm is preferable. In the wet process, the materials forming each layer are dissolved or dispersed in a suitable solvent such as ethanol, chloroform, tetrahydrofuran and dioxane, and a thin film is formed from the solution or the dispersion. Any of the above solvents can be used. Suitable resins and additives may be used in any of the organic thin films to improve the property for film formation and prevent formation of pin holes. Examples of the resin which can be used include insulating resins such as polystyrene, polycarbonates, polyarylates, polyesters, polyamides, polyurethanes, polysulfones, polymethyl methacrylate, polymethyl acrylate and cellulose, copolymers of the insulating resins, photoconductive resins such as poly-N-vinylcarbazoles and polysilanes and electrically conductive resins such as polythiophene and polypyrrol. Examples of the additive include antioxidants, ultraviolet light absorbents and plasticizers.

As described above, an organic EL device exhibiting excellent efficiency of light emission, heat resistance and purity of color and having a long life can be obtained by using the benzothiophene derivative represented by general formula (1) as the material for organic EL devices in the organic thin film layer of the organic EL device.

The organic EL device of the present invention can be utilized as a planar light emitting body such as flat panel displays of wall televisions, a backlight for copiers, printers and liquid crystal displays, a light source for instruments, a display plate and a marking light.

### EXAMPLES

The present invention will be described more specifically with reference to examples in the following. However, the present invention is not limited to the examples.

### Synthesis Example 1 (Synthesis of Compound (H-1))

Compound (H-1) was synthesized in accordance with the reaction route shown in the following.

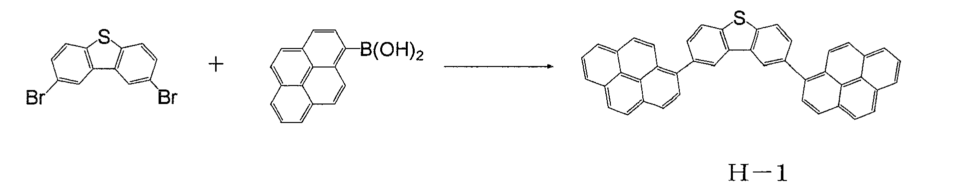

Into a 100 ml three-necked flask, 3.42 g (10 mmole) of 2,8-dibromodibenzothiophene, 5.17g (21 mmole) of pyrene-1-boronic acid and 0.46 g (0.4 mmole, 2% by mole) of tetrakis(triphenylphosphine)-palladium(0) were placed, and the inside of the flask was purged with argon. Into the purged flask, 30 ml of toluene and 15 ml (3 eq) of a 2M aqueous solution of sodium carbonate were added, and the resultant mixture was heated under the refluxing condition in an oil bath of 100°C for 8 hours. After the reaction mixture was left standing for one night, formed precipitates were separated by filtration and purified by washing with toluene, ion-exchanged water and methanol. The yield was 5.33 g.

The obtained compound was analyzed in accordance with FD-MS and identified to be Compound (H-1). The result of the analysis is shown in the following.

FD-MS: calcd. for C₆₃H₄₆=585; found: m/z=585 (M⁺, 100)

### Synthesis Examples 2 to 12 (Syntheses of Compounds (H-2) to (H-12))

Compounds (H-2) to (H-12) were synthesized in accordance with the same procedures as those conducted in Synthesis Example 1 except that the materials shown in the following reaction routes were used in place of 2,8-dibromodibenzothiophene and pyrene-1-boronic acid, and the obtained compounds were analyzed in accordance with FD-MS and identified.

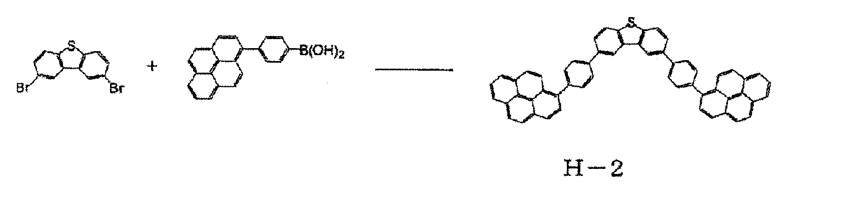

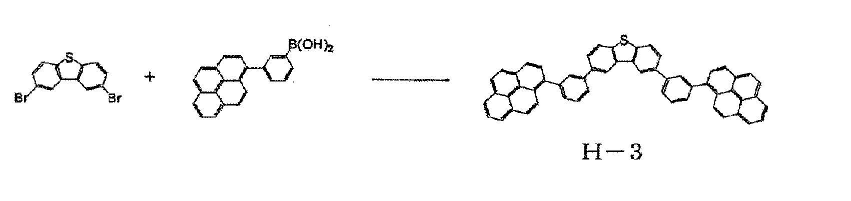

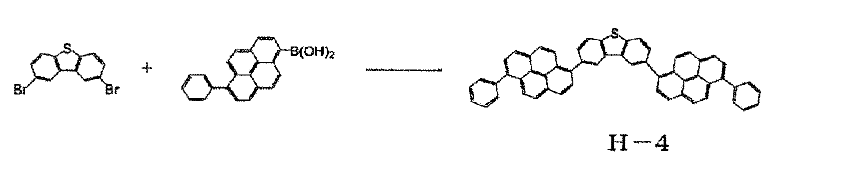

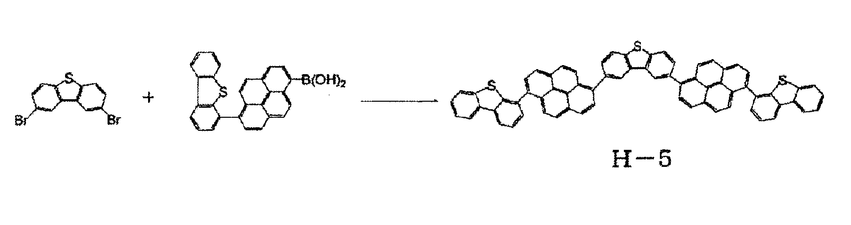

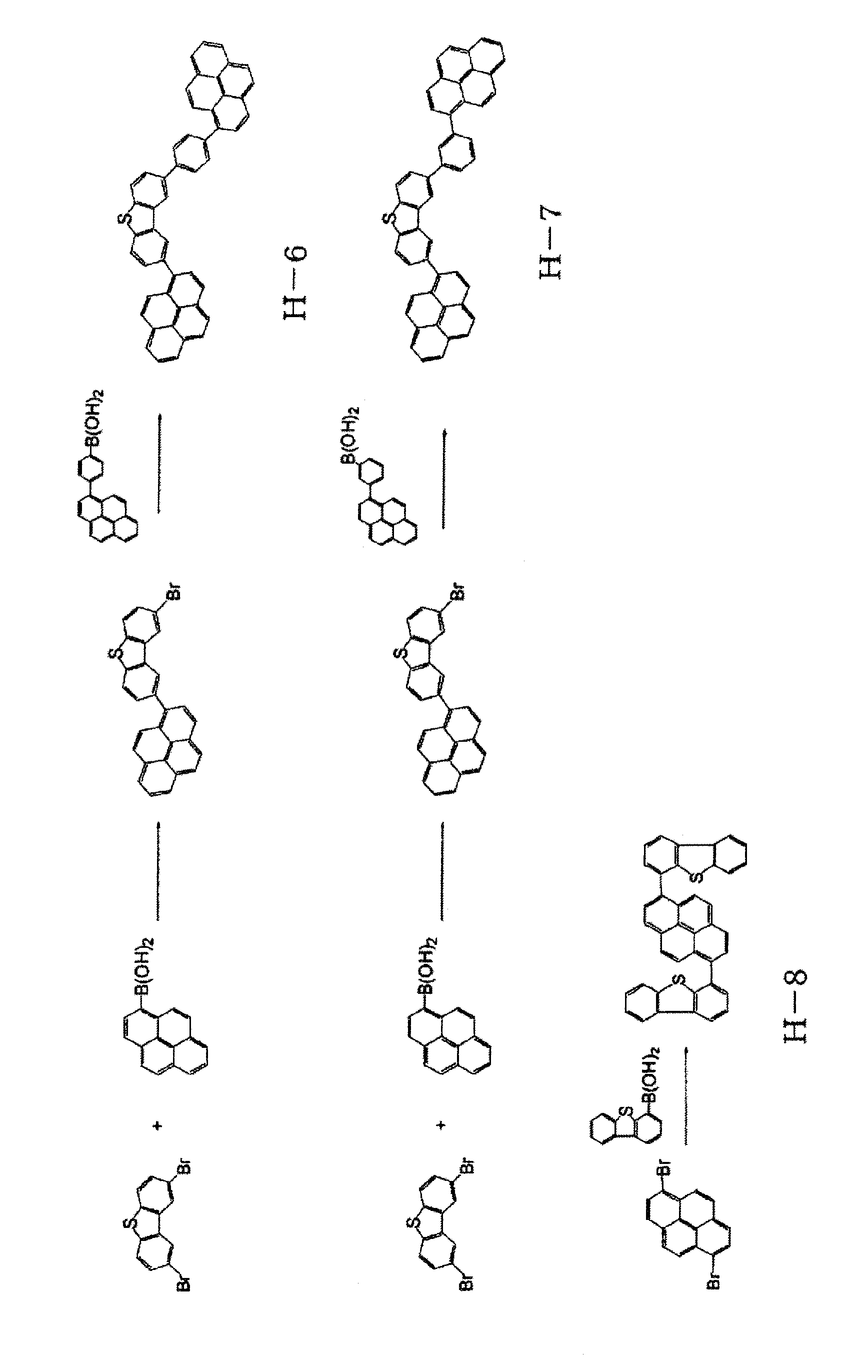

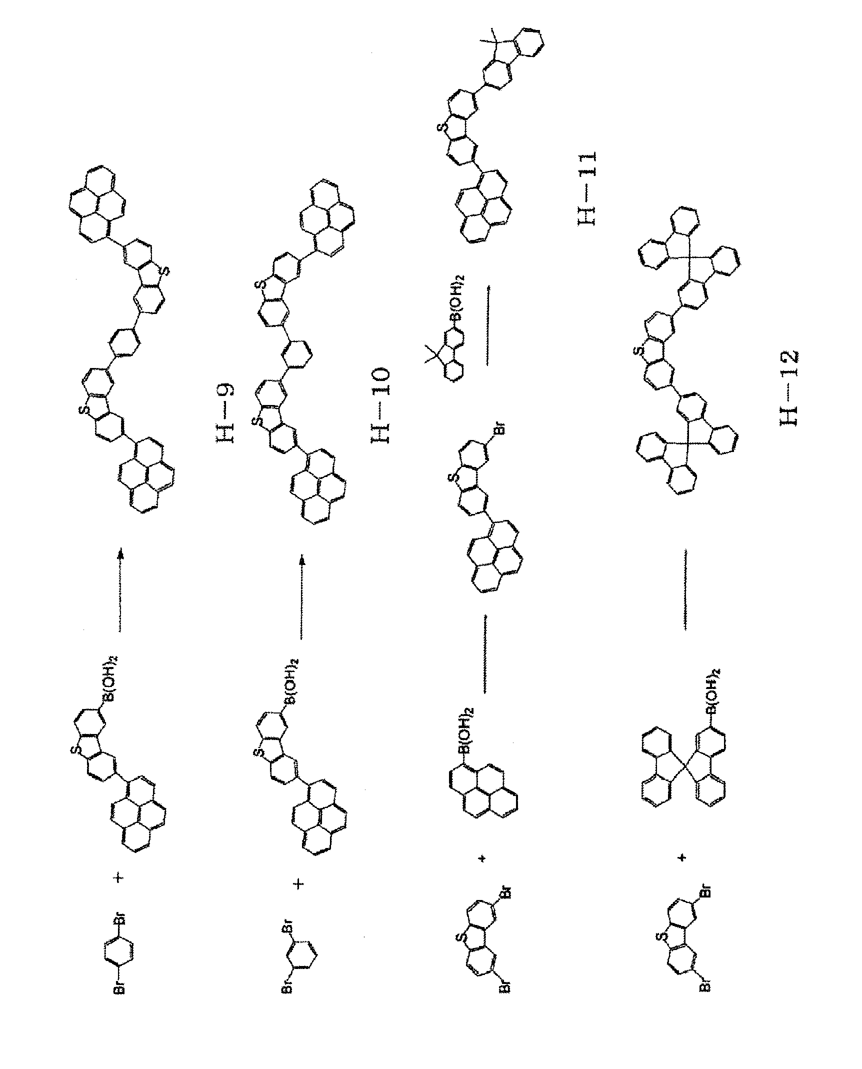

### Example 1 (Preparation and evaluation of an organic EL device)

A glass substrate (manufactured by GEOMATEC Company) of 25 mm × 75 mm × 1.1 mm thickness having an ITO transparent electrode was cleaned by application of ultrasonic wave in isopropyl alcohol for 5 minutes and then by exposure to ozone under irradiation with ultraviolet light for 30 minutes. The cleaned glass substrate having the transparent electrode was attached to a substrate holder of a vacuum vapor deposition apparatus. On the surface of the cleaned substrate at the side having the transparent electrode, a film of N,N'-bis(N,N'-diphenyl-4-aminophenyl)-N,N-diphenyl-4,4'-diamino-1,1'-biphenyl (referred to as a TPD232 film, hereinafter) having a thickness of 60 nm was formed in a manner such that the formed film covered the transparent electrode. The formed TPD232 film worked as the hole injecting layer. On the formed TPD232 film, a layer of N,N,N',N'-tetra(4-biphenyl)diaminobiphenylene (referred to as a TBDB layer, hereinafter) having a thickness of 20 nm was formed. The formed TBDB film worked as the hole transporting layer. On the formed TBDB film, Compound (H-1) as the light emitting material (the host material) was vapor deposited to form a film having a thickness of 40 nm. At the same time, as the light emitting molecule, an amine compound D1 having styryl group, which is shown in the following, was vapor deposited in an amount such that the ratio of the amounts by weight of Compound (H-1) to D1 was 3:40. The formed film worked as the light emitting layer. On the formed film, a film of Alq shown in the following having a thickness of 10 nm was formed. This film worked as the electron injecting layer. Thereafter, LiF (the source of LiF: manufactured by SAES GETTERS Company) as the reducing dopant and Alq were binary vapor deposited, and an Alq:Li film (the thickness: 10 nm) was formed as the electron injecting layer (the cathode). On the formed Alq:Li film, Al metal was vapor deposited to form a metal cathode, and an organic EL device was prepared.

The obtained organic EL device was evaluated with respect to (1) and (2) described in the following, and the results are shown in Table 1.
(1) Initial properties: A prescribed voltage was applied, and the current at the time of application was measured. At the same time, the luminance of the emitted light and the chromaticity coordinates of CIE 1939 were measured using a luminance meter (manufactured by MINOLTA Co., Ltd.; CS-1000), and the efficiency of light emission was calculated. The evaluation was made based on the results.
(2) Life: The obtained device was driven under a constant current at the initial luminance of 1,000 cd/m², and the life was evaluated based on the time when the luminance of the emitted light decreased to one half of the initial value.

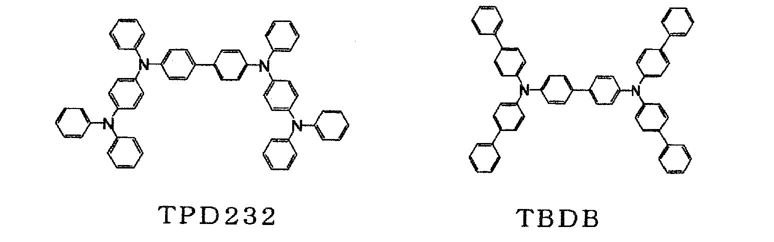

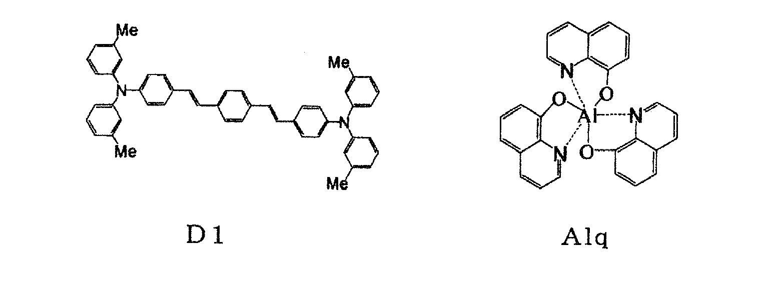

### Examples 2 to 12

Organic EL devices were prepared in accordance with the same procedures as those conducted in Example 1 except that the materials shown in Table 1 were used as the light emitting material (the host material) in place of Compound (H-1) and evaluated in accordance with the same procedures as those conducted in Example 1. The results are shown in Table 1.

### Comparative Examples 1 to 4

Comparative Compounds 1 to 4 were synthesized in accordance with the same procedures as those conducted in Synthesis Example 1 except that the materials shown in the following reaction routes were used in place of 2,8-dibromodibenzothiophene and pyrene-1-boronic acid, and the obtained compounds were analyzed in accordance with FD-MS and identified.

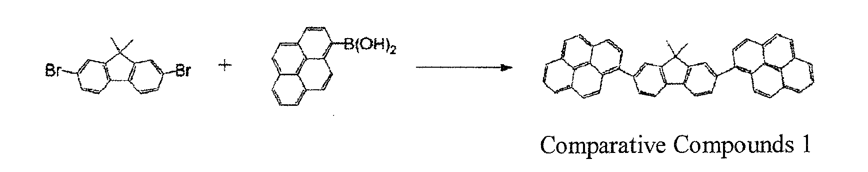

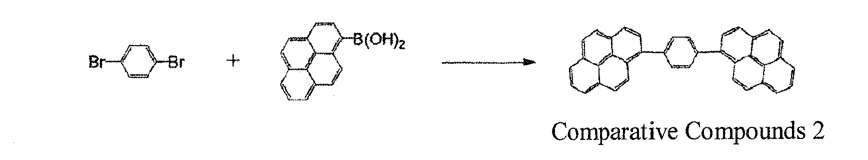

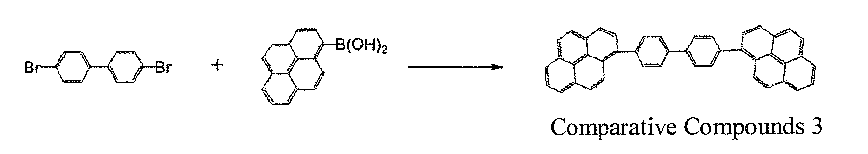

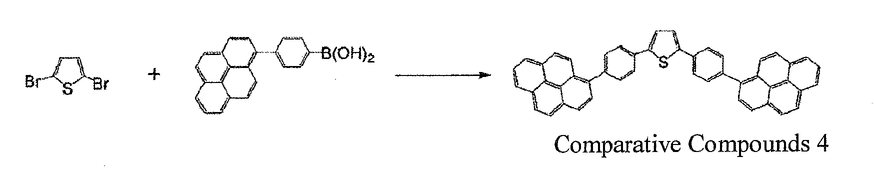

Organic EL devices were prepared in accordance with the same procedures as those conducted in Example 1 except that Comparative Compounds 1 to 4 shown in Table 1 were used as the light emitting material (the host material) in place of Compound (H-1). The prepared organic EL devices were evaluated in accordance with the same procedures as those conducted in Example 1. The results are shown in Table 1.

**Table 1**

| | Light emitting material (host material) | Chromaticity coordinates (CIEx, CIEy) | Efficiency of light emission (cd/A) | Half life (hour) |
|---|---|---|---|---|
| Example 1 | H-1 | (0.14, 0.16) | 11.6 | 9250 |
| Example 2 | H-2 | (0.15, 0.17) | 10.9 | 8980 |
| Example 3 | H-3 | (0.15, 0.16) | 11.2 | 9040 |
| Example 4 | H-4 | (0.15, 0.17) | 11.1 | 8190 |
| Example 5 | H-5 | (0.16, 0.17) | 11.2 | 7760 |
| Example 6 | H-6 | (0.15, 0.18) | 10.7 | 7870 |
| Example 7 | H-7 | (0.14,0.17) | 11.2 | 8860 |
| Example 8 | H-8 | (0.15, 0.18) | 10.7 | 8300 |
| Example 9 | H-9 | (0.16, 0.19) | 12.2 | 7860 |
| Example 10 | H-10 | (0.15, 0.18) | 10.7 | 8300 |
| Example 11 | H-11 | (0.16, 0.19) | 11.8 | 7680 |
| Example 12 | H-12 | (0.16, 0.18) | 10.9 | 8080 |
| Comparative Example 1 | Comparative Compound 1 | (0.17, 0.20) | 8.9 | 3530 |
| Comparative Example 2 | Comparative Compound 2 | (0.17, 0.22) | 6.5 | 4360 |
| Comparative Example 3 | Comparative Compound 3 | (0.26, 0.38) | 7.5 | - |
| Comparative Example 4 | Comparative Compound 4 | (0.22, 0.36) | 6.8 | - |

As shown in Table 1, the organic EL devices of Examples comprising the benzothiophene derivatives in the light emitting layer emitted light having purer blue color, had a longer life and exhibited a more excellent efficiency of light emission than those of the organic EL devices of Comparative Examples using conventional fluorene linker compounds. When the biphenylene and thiophene linker compounds heretofore disclosed were used, the purity of color markedly deteriorated.

### INDUSTRIAL APPLICABILITY

As specifically described in the above, the organic EL device using the benzothiophene derivative of the present invention emits blue light, exhibits a great efficiency of light emission and has a long life and, therefore, is very useful as the organic EL device of a great value in practical applications.

## Claims

1. A benzothiophene derivative represented by following general formula (1):
(A)ₕ-(X)ₖ-(B)ₘ-L-(C)ₙ-(Y)ₚ-(D)_{q} (1)
wherein
X represents a substituted or unsubstituted pyrene residue group;
A and D each independently represent hydrogen atom, a substituted or unsubstituted aromatic hydrocarbon group having 3 to 50 ring carbon atoms, a substituted or unsubstituted aromatic heterocyclic group having 1 to 50 ring carbon atoms, a substituted or unsubstituted alkyl or alkylene group having 1 to 50 carbon atoms or a substituted or unsubstituted alkenyl or alkenylene group having 1 to 50 carbon atoms;
B and C each independently represent a single bond, a substituted or unsubstituted aromatic hydrocarbon group having 3 to 50 ring carbon atoms, a substituted or unsubstituted aromatic heterocyclic group having 1 to 50 ring carbon atoms, a substituted or unsubstituted alkyl or alkylene group having 1 to 50 carbon atoms or a substituted or unsubstituted alkenyl or alkenylene group having 1 to 50 carbon atoms;
Y represents a substituted or unsubstituted condensed cyclic group and/or condensed heterocyclic group having 5 to 50 ring carbon atoms;
L represents a substituted or unsubstituted benzothiophenylene group;
**k** represents an integer of 1 to 3;
**h** and **q** each represent an integer of 0 to 4;
**m** and **n** each represent an integer of 0 to 5; and
p represents an integer of 0 to 3.

2. A benzothiophene derivative according to Claim 1, which is represented by any one of following general formulae (2) and (3):
X-(B)ₘ-L-(C)ₙ-Y (2)
(A)ₕ-(X)ₖ-(B)ₘ-L (3)
wherein X, A, B, C, Y, L, **h, k, m** and n are as defined in Claim 1.

3. A benzothiophene derivative according to Claim 1, which is represented by any one of following general formulae (4) to (7): 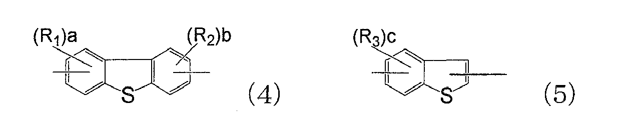 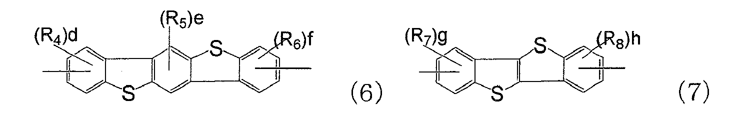 wherein
R₁ to R₈ each independently represent a substituted or unsubstituted aromatic hydrocarbon group having 3 to 50 ring carbon atoms, a substituted or unsubstituted aromatic heterocyclic group having 1 to 50 ring carbon atoms, a substituted or unsubstituted alkyl group having 1 to 50 carbon atoms, a substituted or unsubstituted alkoxyl group having 1 to 50 carbon atoms, a substituted or unsubstituted aralkyl group having 1 to 50 carbon atoms, a substituted or unsubstituted aryloxyl group having 1 to 50 carbon atoms, a substituted or unsubstituted arylthio group having 1 to 50 carbon atoms, a substituted or unsubstituted alkoxycarbonyl group having 1 to 50 carbon atoms, carboxyl group, a halogen atom, cyano group, nitro group or hydroxyl group, and adjacent groups represented by any of R₁ to R₈ may be bonded to each other to form a cyclic structure when a plurality of groups are present;
**a** to **d** and **f** to **h** each represent an integer of 0 to 3; and
e represents an integer of 0 to 2.

4. A benzothiophene derivative according to Claim 1, which is a light emitting material for organic electroluminescence devices.

5. A benzothiophene derivative according to Claim 1, which is a host material for organic electroluminescence devices.

6. An electroluminescence device which comprises a cathode, an anode and an organic thin film layer which comprises at least one layer comprising at least a light emitting layer and is disposed between the cathode and the anode, wherein at least one layer in the organic thin film layer comprises the benzothiophene derivative described in Claim 1 singly or as a component of a mixture.

7. An electroluminescence device according to Claim 6, wherein the light emitting layer comprises the benzothiophene derivative.

8. An electroluminescence device according to Claim 6, wherein the light emitting layer further comprises an arylamine compound.

9. An electroluminescence device according to Claim 6, wherein the light emitting layer further comprises a styrylamine compound.

10. An electroluminescence device according to Claim 6, wherein the light emitting layer further comprises a metal complex compound.
